# EUROPÄISCHE PATENTANMELDUNG

(11) **EP 1 894 942 A1**
(43) Veröffentlichungstag der Anmeldung: **05.03.2008**
(21) Anmeldenummer: 07114897.7
(22) Anmeldetag: 24.08.2007
(51) Int. Cl.: C07K 14/245, C07K 14/255, C12N 1/20, C12P 13/08

(54) **Verfahren zur Herstellung von L-Aminosäuren unter Verwendung von verbesserten Stämmen der Familie Enterobacteriaceae**

(30) Priorität: 01.09.2006 DE 102006041167
(71) Anmelder: Evonik Degussa GmbH, 40474 Düsseldorf (DE)
(72) Erfinder: Schneider, Frank, 33790, Halle (DE)

(57) **Zusammenfassung**

Die Erfindung betrifft ein Verfahren zur Herstellung von L-Aminosäuren durch Fermentation von rekombinanten Mikroorganismen der Familie Enterobacteriaceae, dadurch gekennzeichnet, dass man
a) die die gewünschte L-Aminosäure produzierenden Mikroorganismen, in denen man den ytfF-ORF, für das Genprodukt kodierende Nukleotidsequenzen oder Allele verstärkt, insbesondere überexprimiert, in einem Medium kultiviert unter Bedingungen, bei denen die gewünschte L-Aminosäure im Medium oder in den Zellen angereichert wird, und
b) die gewünschte L-Aminosäure isoliert, wobei gegebenenfalls Bestandteile der Fermentationsbrühe und/oder die Biomasse in ihrer Gesamtheit oder Anteilen (≥ 0 bis 100 %) im isolierten Produkt verbleiben oder vollständig entfernt werden.

## Beschreibung

Diese Erfindung betrifft ein Verfahren zur Herstellung von L-Aminosäuren, insbesondere L-Threonin, unter Verwendung von rekombinanten Mikroorganismen der Familie Enterobacteriaceae, in denen der offene Leserahmen (ORF) mit der Bezeichnung ytfF verstärkt, insbesondere überexprimiert wird, und diese Mikroorganismen.

### Stand der Technik

L-Aminosäuren, insbesondere L-Threonin, finden in der Humanmedizin und in der pharmazeutischen Industrie, in der Lebensmittelindustrie und ganz besonders in der Tierernährung Anwendung.

Es ist bekannt, dass L-Aminosäuren durch Fermentation von Stämmen der Enterobacteriaceae, insbesondere Escherichia coli (E. coli) und Serratia marcescens, hergestellt werden. Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie z.B. die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

In Wildtypstämmen verhindern strikte Regulationsmechanismen die über den Eigenbedarf hinausgehende Produktion von Stoffwechselprodukten wie Aminosäuren und deren Abgabe ins Medium. Die Konstruktion von aus Herstellersicht Aminosäure überproduzierenden Stämmen erfordert deshalb, diese Stoffwechselregulationen zu überwinden.

Zur Beseitigung der Kontrollmechanismen und Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Metabolite sind und L-Aminosäuren wie z.B. L-Threonin produzieren.

Seit einigen Jahren werden ebenfalls Methoden der rekombinanten DNA-Technik zur gezielten Stammverbesserung L-Aminosäuren produzierender Stämme der Familie Enterobacteriaceae eingesetzt, indem man einzelne Aminosäure-Biosynthesegene amplifiziert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2nd edition, ASM Press, Washington, D.C., USA, (1996) zu finden. Eine Zusammenfassung zum Stoffwechsel und zur Produktion von L-Threonin ist veröffentlicht durch Debabov (Advances in Biochemical Engineering Vol.79, 113-136 (2003)).

### Aufgabe der Erfindung

Die Erfinder haben sich die Aufgabe gestellt, neue Maßnahmen zur verbesserten fermentativen Herstellung von L-Aminosäuren, insbesondere L-Threonin, bereitzustellen.

### Beschreibung der Erfindung

Gegenstand der Erfindung sind rekombinante L-Aminosäure produzierende Mikroorganismen der Familie Enterobacteriaceae, die einen verstärkten oder überexprimierten offenen Leserahmen ytfF, der für ein putatives Membranprotein eines Aminosäuretransporters kodiert, oder für dessen Genprodukt kodierende Nukleotidsequenzen enthalten, und die vermehrt L-Aminosäuren, insbesondere L-Threonin, produzieren und in der Zelle oder im Medium anreichern.

Als Ausgangspunkt für den Vergleich dienen jeweils die für den ytfF-ORF nicht rekombinanten Mikroorganismen, die keinen verstärkten ytfF-ORF enthalten und an denen die erfindungsgemäße Verstärkung oder Überexpression nicht durchgeführt wurde.

Zu diesen Mikroorganismen gehören insbesondere Mikroorganismen der Familie Enterobacteriaceae, in denen ein Polynukleotid verstärkt wird, das für ein Polypeptid kodiert, dessen Aminosäuresequenz zu mindestens 70% oder zu mindestens 80% oder mindestens 90%, insbesondere zu mindestens 95%, bevorzugt zu mindestens 98% oder mindestens 99%, besonders bevorzugt zu 99,6% und ganz besonders bevorzugt zu 100% identisch ist mit einer Aminosäuresequenz, ausgewählt aus der Gruppe SEQ ID No. 2 und SEQ ID No. 4.

Die genannten Mikroorganismen enthalten isolierte Polynukleotide, ausgewählt aus der Gruppe:
a) Polynukleotid mit einer Nukleotidsequenz, ausgewählt aus SEQ ID No. 1 oder SEQ ID No. 3 und den dazu komplementären Nukleotidsequenzen;
b) Polynukleotid mit einer Nukleotidsequenz, die der SEQ ID No. 1 oder SEQ ID No. 3 im Rahmen der Degeneration des genetischen Codes entspricht;
c) Polynukleotidsequenz mit einer Sequenz, die mit der zur Sequenz SEQ ID No. 1 oder SEQ ID No. 3 komplementären Sequenz unter stringenten Bedingungen hybridisiert, wobei die stringenten Bedingungen bevorzugt durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1 oder SEQ ID No. 3, die funktionsneutrale Sinnmutanten enthält,
wobei die Polynukleotide für ein putatives Membranprotein eines Aminosäuretransporters kodieren.

Gegenstand der Erfindung ist ebenso ein Verfahren zur fermentativen Herstellung von L-Aminosäuren, insbesondere L-Threonin, unter Verwendung von rekombinanten Mikroorganismen der Familie Enterobacteriaceae, die insbesondere bereits vor der erfindungsgemäßen Verstärkung oder Überexpression L-Aminosäuren produzieren, und in denen mindestens der offene Leserahmen (ORF) mit der Bezeichnung ytfF oder für dessen Genprodukt kodierende Nukleotidsequenzen verstärkt wird bzw. werden.

Bevorzugt werden die beschriebenen Mikroorganismen eingesetzt.

Werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin gemeint. Besonders bevorzugt ist L-Threonin.

Der Begriff "Verstärkung" beschreibt in diesem Zusammenhang die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene, des ORFs bzw. der ORFs um mindestens eine (1) Kopie erhöht, einen starken Promotor funktionell mit dem Gen verknüpft oder ein Gen oder Allel oder ORF verwendet, das für ein entsprechendes Enzym bzw. Protein mit einer hohen Aktivität kodiert und gegebenenfalls diese Maßnahmen kombiniert.

Als offener Leserahmen (ORF, open reading frame) wird ein Abschnitt einer Nukleotidsequenz bezeichnet, der für ein Protein beziehungsweise Polypeptid oder Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Als Genprodukt bezeichnet man im Allgemeinen das von einer Nukleotidsequenz, d.h. einem ORF, einem Gen oder einem Allel kodierte Protein oder die kodierte Ribonukleinsäure.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (parent strain) wird der Mikroorganismus verstanden, an dem die erfindungsgemäße Verstärkung oder Überexpression durchgeführt wird.

Gegenstand der Erfindung ist ein Verfahren zur Herstellung von L-Aminosäuren durch Fermentation von rekombinanten Mikroorganismen der Familie Enterobacteriaceae, dadurch gekennzeichnet, dass man
a) die die gewünschte L-Aminosäure produzierenden Mikroorganismen, in denen man den offenen Leserahmen ytfF oder für dessen Genprodukt kodierende Nukleotidsequenzen oder Allele verstärkt, insbesondere überexprimiert, in einem Medium kultiviert unter Bedingungen, bei denen die gewünschte L-Aminosäure im Medium oder in den Zellen angereichert wird, und,
b) die gewünschte L-Aminosäure isoliert, wobei gegebenenfalls Bestandteile der Fermentationsbrühe und/oder die Biomasse in ihrer Gesamtheit oder Anteilen (≥ 0 bis 100 %) im isolierten Produkt verbleiben oder vollständig entfernt werden.

Die insbesondere rekombinanten Mikroorganismen mit einem verstärkten oder überexprimierten offenen Leserahmen (ORF) mit der Bezeichnung ytfF, die ebenfalls Gegenstand der vorliegenden Erfindung sind, können L-Aminosäuren aus Glucose, Saccharose, Laktose, Fructose, Maltose, Melasse, gegebenenfalls Stärke, gegebenenfalls Cellulose oder aus Glycerin und Ethanol, gegebenenfalls auch aus Mischungen, herstellen. Es handelt sich um Vertreter der Familie Enterobacteriaceae, ausgewählt aus den Gattungen Escherichia, Erwinia, Providencia und Serratia. Die Gattungen Escherichia und Serratia werden bevorzugt. Bei der Gattung Escherichia ist insbesondere die Art Escherichia coli und bei der Gattung Serratia insbesondere die Art Serratia marcescens zu nennen.

Rekombinante Mikroorganismen werden im Allgemeinen durch Transformation, Transduktion oder Konjugation, oder einer Kombination dieser Methoden, mit einem Vektor erzeugt, der den gewünschten ORF, das gewünschte Gen, ein Allel dieses ORFs oder Gens oder Teile davon und/oder einen die Exprimierung des ORFs oder Gens verstärkenden Promotor enthält. Bei diesem Promotor kann es sich um den durch verstärkende Mutation aus der eigenen, upstream des Gens oder ORFs befindlichen regulatorischen Sequenz hervorgegangenen Promotor handeln, oder es wurde ein effizienter Promotor mit dem Gen oder ORF fusioniert.

Als Elternstamm eignen sich Mikroorganismen der Familie Enterobacteriaceae, die insbesondere bereits vor der erfindungsgemäßen Verstärkung oder Überexpression L-Aminosäuren produzieren.

Als zum Elternstamm geeignete, insbesondere L-Threonin produzierende Stämme der Gattung Escherichia, insbesondere der Art Escherichia coli sind beispielsweise zu nennen:
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli TH 14.97 (WO 02/26993)
- Escherichia coli TH 21.97 (WO 02/26993)
- Escherichia coli BKIIM B-3996 (US-A-5,175,107)
- Escherichia coli BKIIM B-3996ΔtdhΔpckA/pVIC40 (WO 02/29080)
- Escherichia coli kat 13 (WO 98/04715)
- Escherichia coli Kat 69.9 (WO 02/26993)
- Escherichia coli KCCM-10132 (WO 00/09660)
- Escherichia coli KCCM-10168 (WO 01/14525)
- Escherichia coli KCCM-10133 (WO 00/09661)

Als zum Elternstamm geeignete L-Threonin produzierende Stämme der Gattung Serratia, insbesondere der Art Serratia marcescens sind beispielsweise zu nennen:
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (Applied Biochemistry and Biotechnology 37(3): 255-265 (1992))

L-Threonin produzierende Stämme aus der Familie der Enterobacteriaceae besitzen bevorzugt, unter anderen, ein oder mehrere der genetischen bzw. phänotypischen Merkmale ausgewählt aus der Gruppe: Resistenz gegen α-Amino-β-Hydroxyvaleriansäure, Resistenz gegen Thialysin, Resistenz gegen Ethionin, Resistenz gegen α-Methylserin, Resistenz gegen Diaminobernsteinsäure, Resistenz gegen α-Aminobuttersäure, Resistenz gegen Borrelidin, Resistenz gegen Cyclopentan-Carboxylsäure, Resistenz gegen Rifampicin, Resistenz gegen Valin-Analoga wie beispielsweise Valinhydroxamat, Resistenz gegen Purinanaloga wie beispielsweise 6-Dimethylaminopurin, Bedürftigkeit für L-Methionin, gegebenenfalls partielle und kompensierbare Bedürftigkeit für L-Isoleucin, Bedürftigkeit für meso-Diaminopimelinsäure, Auxotrophie bezüglich Threonin-haltiger Dipeptide, Resistenz gegen L-Threonin, Resistenz gegen Threonin-Raffinat, Resistenz gegen L-Homoserin, Resistenz gegen L-Lysin, Resistenz gegen L-Methionin, Resistenz gegen L-Glutaminsäure, Resistenz gegen L-Aspartat, Resistenz gegen L-Leucin, Resistenz gegen L-Phenylalanin, Resistenz gegen L-Serin, Resistenz gegen L-Cystein, Resistenz gegen L-Valin, Empfindlichkeit gegenüber Fluoropyruvat, defekte Threonin-Dehydrogenase, gegebenenfalls Fähigkeit zur Saccharose-Verwertung, Verstärkung des Threonin-Operons, Verstärkung der Homoserin-Dehydrogenase I-Aspartatkinase I bevorzugt der feed back resistenten Form, Verstärkung der Homoserinkinase, Verstärkung der Threoninsynthase, Verstärkung der Aspartatkinase, gegebenenfalls der feed back resistenten Form, Verstärkung der Aspartatsemialdehyd-Dehydrogenase, Verstärkung der Phosphoenolpyruvat-Carboxylase, gegebenenfalls der feed back resistenten Form, Verstärkung der Phosphoenolpyruvat-Synthase, Verstärkung der Transhydrogenase, Verstärkung des RhtB-Genproduktes, Verstärkung des RhtC-Genproduktes, Verstärkung des YfiK-Genproduktes, Verstärkung einer Pyruvat-Carboxylase, und Abschwächung der Essigsäurebildung.

Es wurde gefunden, dass Mikroorganismen der Familie Enterobacteriaceae nach Überexpression des Gens oder offenen Leserahmens (ORF) ytfF, oder dessen Allelen, vermehrt L-Aminosäuren, insbesondere L-Threonin, produzieren und in der Zelle oder im Medium anreichern

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminipeptidase) die N-terminale Aminosäure Methionin abgespalten werden kann.

Aus dem ebenfalls zur Familie Enterobacteriaceae gehörenden Salmonella typhimurium ist die Nukleotidsequenz für den ytfF-ORF ebenso bekannt (Accession No.: NC_003197 (Region: 4637134-4638099).

Der ytfF-ORF von Escherichia coli K12 wird unter anderem durch folgende Angaben beschrieben:

Das Genprodukt des ytfF-ORFs ist annotiert als putatives Membranprotein eines Aminosäuretransporters der Familie der DM-Transporter (Jack et al., European Journal of Biochemistry 268 (2001) 3620-3639). Es ist beschrieben als ein Mitglied der rhtA-Familie (Livshits et al., Research in Microbiology 154 (2003) 123-135).

Accession No.: U00096 (Region: 4430114-4431088) Alternativer Genname: b4210

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber sind die bekannte Sequenz zum ytfF-ORF von Escherichia coli unter der SEQ ID No. 1 und die bekannten Sequenz zum ytfF-ORF von Salmonella typhimurium unter der SEQ ID No. 3 dargestellt. Die von diesen Leserahmen kodierten Proteine sind als SEQ ID No. 2 und SEQ ID No. 4 dargestellt.

Die in den angegebenen Textstellen beschriebenen offenen Leserahmen können erfindungsgemäß verwendet werden. Weiterhin können Allele der Gene oder offene Leserahmen verwendet werden, die sich aus der Degeneriertheit des genetischen Codes oder durch funktionsneutrale Sinnmutationen ("sense mutations") ergeben. Die Verwendung endogener Gene bzw. endogener offener Leserahmen wird bevorzugt.

Unter "endogenen Genen" oder "endogenen Nukleotidsequenzen" versteht man die in der Population einer Art vorhandenen Gene oder offene Leserahmen oder Allele beziehungsweise Nukleotidsequenzen.

Zu den Allelen des ytfF-ORFs, welche funktionsneutrale Sinnmutationen enthalten, zählen unter anderem solche, die zu höchstens 40 oder zu höchstens 30 oder zu höchstens 20, bevorzugt zu höchstens 10 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 40, 30, 20, 10, 5, 3 oder 2 Aminosäuren oder Aminosäurereste.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartige als Indels bezeichneter Veränderungen kann 2, 3, 5, 10 in keinem Falle aber mehr als 20 Aminosäuren betreffen.

Zu den geeigneten Allelen gehören ferner solche die durch Hybridisierung, insbesondere unter stringenten Bedingungen unter Verwendung von SEQ ID No. 1 oder SEQ ID No. 3 oder Teilen davon insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID No. 1 oder SEQ ID No. 3 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Zur Erzielung einer Verstärkung können beispielsweise die Expression der Gene oder offenen Leserahmen oder Allele oder die katalytischen Eigenschaften des Proteins erhöht werden. Gegebenenfalls können beide Maßnahmen kombiniert werden.

Zur Erzielung einer Überexpression kann beispielsweise die Kopienzahl der entsprechenden Gene oder offenen Leserahmen erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Threonin-Produktion zu steigern, des weiteren vorteilhaft sein kann auch die Verwendung von Promotoren zur Genexpression, die eine andere zeitliche Genexpression ermöglicht. Auf der Ebene der translationalen Regulation der Genexpression ist es möglich, die Häufigkeit der Initiation (Anlagerung des Ribosoms an die m-RNA) oder die Geschwindigkeit der Elongation (Verlängerungsphase) zu erhöhen. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die ORFs, Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0332448 beschrieben, oder der Phage lambda (λ) verwendet werden. Eine Erhöhung der Kopienzahl kann auch dadurch erzielt werden, dass eine weitere Kopie in eine weitere Stelle des Chromosoms - beispielsweise in die att-site des Phagen λ (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, 1pp-Promotor oder P_{L}-Promotor und P_{R}-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren, der nar-Promotor oder die Promotoren der Gene rrsG, rnpB, csrA, csrB, ompA, fusA, pepQ, rplX oder rpsG verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression plasmidgebundener Gene kontrollieren (Glascock und Weickert, Gene 223, 221-231 (1998)). Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist. Der Austausch des Promotors gegen einen zum Beispiel in genomweit-vergleichenden Expressionsanalysen ermittelten Promotor mit gleichmäßig hoher Expression im Verlauf eines Fermentationsprozesses bewirkt eine gleichmäßige Verstärkung. Eine andere zeitliche Genexpression kann beispielsweise wie bei Walker et al. (Journal of Bacteriology 181: 1269-80 (1999)) beschrieben durch die Verwendung des Wachstumsphasenabhängigen fis Promotors erreicht werden. Die Geschwindigkeit der Elongation wird durch die Codon-Verwendung beeinflusst, durch den Gebrauch von Codons für im Elternstamm (parent strain) häufig vorkommenden t-RNAs kann die Genexpression verstärkt werden. Desweiteren kann der Austausch eines Start-Codons zu dem in Escherichia coli mit 77% am häufigsten vorkommenden Codon ATG die Translation erheblich verbessern, da das Codon AUG zweibis dreimal effektiver ist als zum Beispiel die Codons GUG und UUG (Khudyakov et al., FEBS Letters 232(2):369-71 (1988); Reddy et al., Proceedings of the National Academy of Sciences of the USA 82(17):5656-60 (1985)). Auch die Sequenzumgebung des Start-Codons kann optimiert werden, da zusammenwirkende Effekte zwischen dem Start-Codon und den flankierenden Bereichen beschrieben sind (Stenstrom et al., Gene 273(2):259-65 (2001); Hui et al., EMBO Journal 3(3) : 623-9 (1984)).

Allgemeine Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie z.B. von pACYC184 abgeleitete Kloniervektoren (Bartolomé et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. In einem erfindungsgemäßen Verfahren kann man einen mit einem Plasmidvektor transformierten Stamm einsetzen, wobei der Plasmidvektor mindestens den ytfF-ORF, oder für dessen Genprodukt kodierende Nukleotidsequenzen oder Allele trägt.

Unter dem Begriff Transformation versteht man die Aufnahme einer isolierten Nukleinsäure durch einen Wirt (Mikroorganismus).

Es ist ebenfalls möglich, Mutationen, welche die Expression der jeweiligen Gene oder offenen Leserahmen betreffen, durch Sequenzaustausch (Hamilton et al.; Journal of Bacteriology 171: 4617-4622 (1989)), Konjugation oder Transduktion in verschiedene Stämme zu überführen.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Weiterhin kann es für die Produktion von L-Aminosäuren, insbesondere L-Threonin mit Stämmen der Familie Enterobacteriaceae vorteilhaft sein, zusätzlich zur Verstärkung des offenen Leserahmens ytfF, ein oder mehrere Enzyme des bekannten Threonin-Biosyntheseweges oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

So können beispielsweise gleichzeitig eines oder mehrere der Gene, ausgewählt aus der Gruppe
• mindestens ein Gen des für die Aspartatkinase, die Homoserin-Dehydrogenase, die Homoserinkinase und die Threoninsynthase kodierenden thrABC-Operons (US-A-4,278,765),
• das für die Pyruvat-Carboxylase kodierende pyc-Gen von Corynebacterium glutamicum (WO 99/18228),
• das für die Phosphoenolpyruvat-Synthase kodierende pps-Gen (Molecular and General Genetics 231(2): 332-336 (1992); WO 97/08333),
• das für die Phosphoenolpyruvat-Carboxylase kodierende ppc-Gen (WO 02/064808),
• die für die Untereinheiten der Transhydrogenase kodierenden Gene pntA und pntB (European Journal of Biochemistry 158: 647-653 (1986); WO 95/11985),
• das für das Threoninresistenz vermittelnde Protein kodierende Gen rhtC (EP-A-1 013 765),
• das für das Threonin-Export-Carrier-Protein kodierende thrE-Gen von Corynebacterium glutamicum (WO 01/92545),
• das für die Glutamat-Dehydrogenase kodierende gdhA-Gen (Nucleic Acids Research 11: 5257-5266 (1983); Gene 23: 199-209 (1983); DE19907347),
• das für die Phosphohistidin-Protein-Hexose-Phosphotransferase des Phosphotransferase-Systems PTS kodierende ptsH-Gen des ptsHIcrr-Operons (WO 03/004674),
• das für das Enzym I des Phosphotransferase-Systems PTS kodierende ptsI-Gen des ptsHIcrr-Operons (WO 03/004674),
• das für die Glucose-spezifische IIA Komponente des Phosphotransferase-Systems PTS kodierende crr-Gen des ptsHIcrr-Operons (WO 03/004674),
• das für die Glucose-spezifische IIBC Komponente kodierende ptsG-Gen (WO 03/004670),
• das für die Cystein-Synthase A kodierende cysK-Gen (WO 03/006666),
• das für den Regulator des cys-Regulons kodierende cysB-Gen (WO 03/006666),
• das für das Flavoprotein der NADPH-Sulfit-Reduktase kodierende cysJ-Gen des cysJIH-Operons (WO 03/006666),
• das für das Hämoprotein der NADPH-Sulfit-Reduktase kodierende cysI-Gen des cysJIH-Operons (WO 03/006666),
• das für die Adenylylsulfat-Reduktase kodierende cysH-Gen des cysJIH-Operons (WO 03/006666),
• das für die Decarboxylase Untereinheit der 2-Ketoglutarat Dehydrogenase kodierende sucA-Gen des sucABCD-Operons (WO 03/008614),
• das für die Dihydrolipoyltranssuccinase E2 Untereinheit der 2-Ketoglutarat Dehydrogenase kodierende sucB-Gen des sucABCD-Operons (WO 03/008614),
• das für die β-Untereinheit der Succinyl-CoA Synthetase kodierende sucC-Gen des suc ABCD-Operons (WO 03/008615),
• das für die α-Untereinheit der Succinyl-CoA Synthetase kodierende sucD-Gen des sucABCD-Operons (WO 03/008615),
• das Genprodukt des offenen Leserahmens (ORF) yibD von Escherichia coli (Accession Number AE000439 des National Center for Biotechnology Information (NCBI, Bethesda, MD, USA), DE102004005836.9),
• das Genprodukt des offenen Leserahmens (ORF) yjcG von Escherichia coli (Accession Number NC000913 (Region 4281276-4282925) des National Center for Biotechnology Information (NCBI, Bethesda, MD, USA),
• das Genprodukt des offenen Leserahmens (ORF) ytfR von Escherichia coli (Accession Number NC000913 (Region 4449081-4450583) des National Center for Biotechnology Information (NCBI, Bethesda, MD, USA), der auch unter der Bezeichnung ytfS-ORF bekannt ist,
• das Genprodukt des offenen Leserahmens (ORF) ytfT von Escherichia coli (Accession Number NC000913 (Region 4450594-4451619) des National Center for Biotechnology Information (NCBI, Bethesda, MD, USA),
• das Genprodukt des offenen Leserahmens (ORF) yjfF von Escherichia coli (Accession Number NC000913 (Region 4451630-4452601) des National Center for Biotechnology Information (NCBI, Bethesda, MD, USA), und
• das Genprodukt des offenen Leserahmens (ORF) ytfQ von Escherichia coli (Accession Number NC000913 (Region 4447985-4448941) des National Center for Biotechnology Information (NCBI, Bethesda, MD, USA)
verstärkt, insbesondere überexprimiert werden.

Weiterhin kann es für die Produktion von L-Aminosäuren, insbesondere L-Threonin vorteilhaft sein, zusätzlich zur Verstärkung des offenen Leserahmens ytfF, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

So können beispielsweise gleichzeitig eines oder mehrere der Gene, ausgewählt aus der Gruppe
• das für die Threonin-Dehydrogenase kodierende tdh-Gen (Journal of Bacteriology 169: 4716-4721 (1987)),
• das für die Malat-Dehydrogenase (E.C. 1.1.1.37) kodierende mdh-Gen (Archives in Microbiology 149: 36-42 (1987)),
• das Genprodukt des offenen Leserahmens (ORF) yjfA von Escherichia coli (Accession Number AAC77180 des National Center for Biotechnology Information (NCBI, Bethesda, MD, USA), WO 02/29080),
• das Genprodukt des offenen Leserahmens (ORF) ytfP von Escherichia coli (Accession Number AAC77179 des National Center for Biotechnology Information (NCBI, Bethesda, MD, USA), WO 02/29080),
• das für das Enzym Phosphoenolpyruvat-Carboxykinase kodierende pckA-Gen (WO 02/29080),
• das für die Pyruvat-Oxidase kodierende poxB-Gen (WO 02/36797),
• das für den DgsA-Regulator des Phosphotransferase-Systems kodierende dgsA-Gen (WO 02/081721), das auch unter der Bezeichnung mlc-Gen bekannt ist,
• das für den Fructose-Repressor kodierende fruR-Gen (WO 02/081698), das auch unter der Bezeichnung cra-Gen bekannt ist,
• das für den Sigma³⁸-Faktor kodierende rpoS-Gen (WO 01/05939), das auch unter der Bezeichnung katF-Gen bekannt ist und
• das für die Aspartat Ammonium-Lyase kodierende aspA-Gen (WO 03/008603)
abgeschwächt, insbesondere ausgeschaltet oder die Expression verringert werden.

Der Begriff "Abschwächung" beschreibt in diesem Zusammenhang die Verringerung oder Ausschaltung der intrazellulären Aktivität oder Konzentration eines oder mehrerer Enzyme bzw. Proteine in einem Mikroorganismus, die durch die entsprechende DNA kodiert werden, indem man beispielsweise einen schwächeren Promotor als im für das entsprechende Enzym bzw. Protein nicht rekombinanten Mikroorganismnus oder Elternstamm oder ein Gen bzw. Allel verwendet, das für ein entsprechendes Enzym bzw. Protein mit einer niedrigen Aktivität kodiert bzw. das entsprechende Enzym bzw. Protein oder den offenen Leserahmen oder das Gen inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die erfindungsgemäße Abschwächung oder Ausschaltung durchgeführt wird.

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)), Kawano et al. (Nucleic Acids Research 33(19), 6268-6276 (2005)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservative Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität. Die Verringerung der Genexpression durch Suppression einer Stop-Kodon-Mutation im Kodierbereich durch geeignete t-RNA-Suppressoren ist in der WO 03/074719 beschrieben.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Geeignete Mutationen in den Genen können durch Gen- bzw. Allelaustausch in geeignete Stämme eingebaut werden.

Eine gebräuchliche Methode ist die von Hamilton et al. (Journal of Bacteriology 171: 4617-4622 (1989)) beschriebene Methode des Genaustauschs mit Hilfe eines konditional replizierenden pSC101-Derivates pMAK705. Andere im Stand der Technik beschriebene Methoden wie beispielsweise die von Martinez-Morales et al. (Journal of Bacteriology 181: 7143-7148 (1999)) oder die von Boyd et al. (Journal of Bacteriology 182: 842-847 (2000)) können gleichfalls benutzt werden.

Es ist ebenfalls möglich, Mutationen in den jeweiligen Genen oder Mutationen, die die Expression der jeweiligen Gene oder offenen Leserahmen betreffen, durch Konjugation oder Transduktion in verschiedene Stämme zu überführen.

Gegebenenfalls können die Maßnahmen zur Verstärkung und zur Abschwächung beliebig kombiniert werden.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den nicht rekombinanten Mikroorganismus oder Elternstamm bzw. den Fermentationsprozess unter Verwendung desselben verbessert.

Die erfindungsgemäß hergestellten Mikroorganismen können im batch - Verfahren (Satzkultivierung), im fed batch (Zulaufverfahren), im repeated fed batch-Verfahren (repetitives Zulaufverfahren) oder in einem kontinuierlichen Verfahren (DE102004028859.3 oder US5,763,230) kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 25°C bis 45°C und vorzugsweise bei 30°C bis 40°C. Die Kultur wird solange fortgesetzt, bis sich ein Maximum an L-Aminosäuren bzw. L-Threonin gebildet hat. Dieses Ziel wird normalerweise innerhalb von 10 Stunden bis 160 Stunden erreicht.

Aus der entnommenen Kulturbrühe können die L-Aminosäuren gewonnen, gesammelt oder konzentriert und gegebenenfalls gereinigt werden.

Es ist ebenfalls möglich aus der entnommenen Kulturbrühe (= Fermentationsbrühe) ein Produkt herzustellen, indem man die in der Kulturbrühe enthaltene Biomasse des Bakteriums vollständig (100%) oder nahezu vollständig d.h. mehr als oder größer als (>) 90% entfernt und die übrigen Bestandteile der Fermentationsbrühe weitgehend d.h. zu 30% - 100%, bevorzugt größer gleich (≥) 50%, ≥70% oder ≥90% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder GranulierVerfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10%, kleiner als 5% beträgt.

Die Analyse von L-Aminosäuren kann durch Anionenaustauschchromatographie mit anschließender Ninhydrin Derivatisierung erfolgen, so wie bei Spackman et al. (Analytical Chemistry 30: 1190-1206 (1958)) beschrieben, oder sie kann durch reversed phase HPLC erfolgen, so wie bei Lindroth et al. (Analytical Chemistry 51: 1167-1174 (1979)) beschrieben.

Das erfindungsgemäße Verfahren dient zur fermentativen Herstellung von L-Aminosäuren, wie beispielsweise L-Threonin, L-Isoleucin, L-Valin, L-Methionin, L-Homoserin, L-Tryptophan und L-Lysin, insbesondere L-Threonin.

Die vorliegende Erfindung wird im Folgenden unter Verwendung von Arbeitsbeispielen ausführlicher erläutert.

Die Minimal- (M9) und Universalmedien (LB) für E. coli, die verwendet wurden, sind von J. H. Miller (A Short Course in Bacterial Genetics (1992), Cold Spring Harbor Laboratory Press) beschrieben. Die Isolierung von Plasmid-DNA aus E. coli sowie alle Techniken, die die Restriktion, Ligation, Klenow- und alkalische Phosphatase-Behandlung betreffen, wurden wie von Sambrook et al. beschrieben (Molecular Cloning. A Laboratory Manual (1989), Cold Spring Harbor Laboratory Press) durchgeführt. Die Transformation von E. coli wurde, wenn nicht anders beschrieben, wie bei Chung et al. (Proceedings of the National Academy of Sciences of the United States of America 86: 2172 - 2175 (1989)) beschrieben durchgeführt.

Die Inkubationstemperatur für die Herstellung von Stämmen und Transformanten war 37°C, wenn nicht anders beschrieben.

### Beispiel 1

### Konstruktion des Expressionsplasmids pMW218ytfF

Der ytfF-ORF aus E. coli K12 wurde unter Verwendung der Polymerase-Kettenreaktion (PCR) sowie synthetischer Oligonukleotide amplifiziert. Ausgehend von der Nukleotidsequenz des ytfF-Gens in E. coli K12 MG1655 (SEQ ID No. 1, Accession Number bzw. Zugangsnummer U00096 (Region: 4430114-4431088)) wurden PCR-Primer synthetisiert (Invitrogen, Karlsruhe, Deutschland). Die Primer enthielten Sequenzen für Restriktionsenzyme, welche in der unten gezeigten Nukleotidsequenz durch Unterstreichen markiert sind. Die Primer enthalten die Restriktionsspaltungsstelle für BamHI:
ytfF fw:
   5' - CAGTACGGATCCGGATTGGTCGCGATAAGCCATAGC -3' (SEQ ID No. 5)
ytfF rev:
   5' - TAGTCAGGATCCGGATTCTGACGTTGGCGCATCGAA - 3' (SEQ ID No. 6)

Die für die PCR eingesetzte chromosomale E. coli K12 MG1655 DNA wurde den Anweisungen des Herstellers gemäß mit "Qiagen Genomic-tips 100/G" (QIAGEN, Hilden, Deutschland) isoliert. Ein DNA-Fragment, ca. 1 352 bp groß, konnte mit den spezifischen Primern unter Standard-PCR-Bedingungen (Innis et al. (1990) PCR Protocols. A Guide to Methods and Applications, Academic Press) mit der Vent-DNA-Polymerase (New England BioLabs, Frankfurt, Deutschland) amplifiziert werden (SEQ ID No. 7).

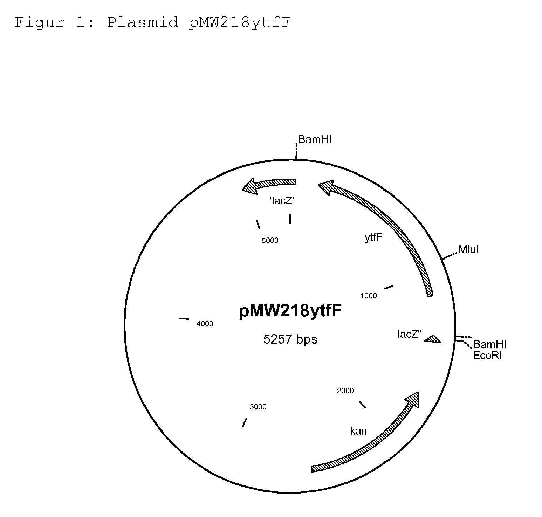

Das amplifizierte ytfF-Fragment wurde mit dem Restriktionsenzym BamHI geschnitten und nach der Reinigung (Purification Kit, QIAGEN, Hilden, Deutschland) in einem 0,8%-igen Agarosegel überprüft. Der Vektor pMW218 (Nippon Gene, Toyama, Japan) wurde mit dem Enzym BamHI gespalten und eine Ligation wurde mit dem geschnittenen ytfF-Fragment durchgeführt. Der E. coli-Stamm TOP10 One Shot® (TOPO TA Cloning Kit, Invitrogen, Groningen, Niederlande) wurde mit dem Ligationsansatz transformiert und Plasmid-tragende Zellen wurden auf LB-Agar selektioniert, zu welchem 50 µg/ml Kanamycin zugegeben wurden. Eine erfolgreiche Klonierung konnte nach der Isolierung der Plasmid-DNA über eine Kontrollspaltung mit den Enzymen EcoRI und MluI gezeigt werden. Das Plasmid wird als pMW218ytfF (Figur 1) bezeichnet.

### Beispiel 2

### Herstellung von L-Threonin mit dem Stamm MG442/pMW218ytfF

Der L-Threonin produzierende E. coli-Stamm MG442 ist in der Patentschrift US-A-4 278 765 beschrieben und als CMIM B-1628 bei der Russischen Nationalen Sammlung für Industrielle Mikroorganismen (Russian National Collection for Industrial Microorganisms) (VKPM, Moskau, Russland) und als DSM 16574 bei der Deutschen Sammlung für Mikroorganismen und Zellkulturen (DSMZ, Braunschweig, Deutschland) gemäß dem Budapester Vertrag hinterlegt.

Der Stamm MG442 wurde mit dem in Beispiel 1 beschriebenen Expressionsplasmid pMW218ytfF und mit dem Vektor pMW218 transformiert, und Plasmid tragende Zellen wurden auf LB-Agar mit 50 µg/ml Kanamycin selektiert. Erfolgreiche Transformationen konnten nach der Isolierung von Plasmid-DNA über Kontrollspaltungen mit den Enzymen MluI und EcoRI nachgewiesen werden. Auf diese Weise bildeten sich die Stämme MG442/pMW218ytfF und MG442/pMW218. Selektierte einzelne Kolonien wurden dann auf Minimalmedium mit der folgenden Zusammensetzung: 3,5 g/l Na₂HPO₄*2H₂O, 1,5 g/l KH₂PO₄, 1 g/l NH₄Cl, 0,1 g/l MgSO₄*7H₂O, 2 g/l Glucose, 20 g/l Agar und 50 mg/l Kanamycin, weiter vermehrt. Die Bildung von L-Threonin wurde in Batch-Kulturen von 10 ml überprüft, die in 100 ml Erlenmeyerkolben enthalten waren. Dazu wurden 10 ml des Vorkulturmediums der folgenden Zusammensetzung: 2 g/l Hefeextrakt, 10 g/l (NH₄)₂SO₄, 1 g/l KH₂PO₄, 0,5 g/l MgSO₄*7H₂O, 15 g/l CaCO₃, 20 g/l Glucose und 50 mg/l Kanamycin, angeimpft, und der Batch bzw. Ansatz wurde 16 Stunden lang bei 37°C und 180 U/min auf einem ESR-Inkubator von der Kühner AG (Birsfelden, Schweiz) inkubiert. 250 µl dieser Vorkultur wurden in 10 ml Produktionsmedium (25 g/l (NH₄)₂SO₄, 2 g/l KH₂PO₄, 1 g/l MgSO₄*7H₂O, 0,03 g/l FeSO₄*7H₂O, 0,018 g/l MnSO₄*1H₂O, 30 g/l CaCO₃, 20 g/l Glucose) überimpft, und der Batch wurde 48 Stunden lang bei 37°C inkubiert. Nach der Inkubation wurde die optische Dichte (OD) der Kultursuspension mit einem LP2W-Photometer von Dr. Lange (Düsseldorf, Deutschland) bei einer Messwellenlänge von 660 nm bestimmt.

Die Konzentration an gebildetem L-Threonin wurde dann im steril filtrierten Kulturüberstand mit einem Aminosäureanalysator von Eppendorf-BioTronik (Hamburg, Deutschland) durch Ionenaustauschchromatographie und Nachsäulen-Reaktion mit Ninhydrindetektion bestimmt.

Das Ergebnis des Experiments ist in Tabelle 1 dargestellt.

**Tabelle 1**

| Stamm | OD (660 nm) | L-Threonin g/l |
|---|---|---|
| MG442/pMW218 | 6,2 | 1,5 |
| MG442/pMW218ytfF | 5,8 | 1,9 |

### Beispiel 3

### Positionsspezifische Mutagenese des thrB-Gens beim E. coli-Stamm MG442

Der L-Homoserin produzierende E. coli-Stamm MG442ΔthrB stammt vom Stamm MG442, der in der Patentschrift US-A-4 278 765 beschrieben ist, und der als CMIM B-1628 bei der Russischen Nationalen Sammlung für Industrielle Mikroorganismen (VKPM, Moskau, Russland) eingereicht wurde.

### 3.1 Konstruktion der Deletionsmutation des thrB-Gens durch Gen-SOEing

Auf der Grundlage der Sequenz des thrB-Gens, welche für E. coli K12 MG1655 (SEQ ID No. 9, Zugangsnummer U00096) bekannt ist, wurden die folgenden Primer-Oligonukleotide für die Erzeugung der thrB-Deletionsmutation mit Hilfe der Polymerasekettenreaktion (PCR) durch das Gen-SOEing-Verfahren (Gene Splicing by Overlap Extension, Horton, Molecular Biotechnology 3: 93 - 98 (1995)) ausgewählt:
thrB_No: 5' - CAGTACGCGGCCGCTGCAGGTGATGAATTGATGAAG - 3' (SEQ ID No. 11)
thrB_Ni: 5' - CACGCAATAACCTTCACACTCCAAATTTATAACTAACCATGTCAGACTCCTAACT - 3' (SEQ ID No. 12)
thrB_Ci: 5' - GTTATAAATTTGGAGTGTGAAGGTTATTGCGTGCGACCTTGTTCGCTCTGTGT - 3' (SEQ ID No. 13)
thrB_Co: 5' - TAGTCGCGGCCGCGCCTCGTGGATAGAGGATAACC - 3' (SEQ ID No. 14)

Die gezeigten Primer wurden von Invitrogen (Karlsruhe, Deutschland) synthetisiert und die PCR-Reaktion wurde durch das Standard-PCR-Verfahren von Innis et al. (PCR Protocols. A Guide to Methods and Applications, 1990, Academic Press) durchgeführt. Die für die PCR verwendete chromosomale E. coli K12 MG1655 DNA wurde gemäß den Anweisungen des Herstellers unter Verwendung von "Qiagen Genomic-tips 100/G" (QIAGEN, Hilden, Deutschland) isoliert. Die Primer thrB_No und thrB_Co enthielten jeweils die Sequenz für eine Spaltungsstelle der Restriktions-Endonuclease NotI, welche durch Unterstreichen in der oben gezeigten Nukleotidsequenz markiert ist. Der Primer thrB_Ni (SEQ ID No. 12) ist aus zwei Regionen zusammengesetzt, von der eine in der Sequenz des thrB-Gens an die Nukleotide 579 bis 600 (SEQ ID No. 9) bindet, und die andere, die 5'-terminale Region des Primers thrB_Ni, eine 33 bp große Nonsense-Ersatzsequenz, entgegengesetzt komplementär zur 5'-terminalen Region des Primers thrB_Ci ist und an die Nukleotide 1 bis 33 des Primers thrB_Ci (SEQ ID No. 13) bindet. Die zweite Region des Primers thrB_Ci bindet in der Sequenz des thrB-Gens an die Nukleotide 1384 bis 1403 (SEQ ID No. 9). Zwischen beiden Bindungs-Regionen in der thrB-Gensequenz sind 783 Basenpaare der kodierenden Sequenz des thrB-Gens deletiert.

Mit Hilfe der PCR ermöglichen die Primer thrB_No und thrB_Ni die Amplifikation eines DNA-Fragments von etwa 0,65 kb Länge, welches die 5'-terminale Region des thrB-Gens und die stromaufwärts gelegene Region enthält, und die Primer thrB_Ci und thrB_Co ermöglichen die Amplifikation eines DNA-Fragments von etwa 0,67 kb Länge, welches die 3'-terminale Region des thrB-Gens und die stromabwärts gelegene Region enthält. Die Amplifikate wurden durch eine nachfolgende Agarose-Gelelektrophorese in einem 0,8%-igem Agarosegel untersucht, aus dem Gel isoliert und durch herkömmliche Methoden (High Pure PCR Product Purification Kit, Roche Diagnostics GmbH, Mannheim, Deutschland) aufgereinigt. Beide Amplifikate wurden zusammen als Matrize bei einer weiteren PCR-Reaktion unter Verwendung der Primer thrB_No und thrB_Co eingesetzt. Dies führte zu der Erzeugung der thrB-Deletionskassette, etwa 1282 bp groß, die das thrB-Gen einschließlich einer 783 bp großen Deletion und einer 33 bp großen Nonsense-Ersatzsequenz, etwa 600 bp der stromaufwärts gelegenen Region des thrB-Gens und etwa 480 bp der stromabwärts gelegenen Region des thrB-Gens enthielt.

Die thrB-Deletionskassette wurde mit der Restriktions-Endonuclease NotI gespalten und durch Agarose-Gelelektrophorese in einem 0,8%-igem Agarosegel identifiziert. Das etwa 1,26 kb lange DNA-Fragment wurde aus dem Gel isoliert, mit herkömmlichen Methoden (High Pure PCR Product Purification Kit, Roche Diagnostics GmbH, Mannheim, Deutschland) aufgereinigt und für eine Ligation mit dem von Link et al. (Journal of Bacteriology 179: 6228 - 6237 (1997)) beschriebenen Klonierungs-Vektor pK03 eingesetzt. Der Vektor pK03 wurde im Voraus mit der Restriktions-Endonuclease NotI gespalten und mit alkalischer Phosphatase (Boehringer, Mannheim) dephosphoryliert, anschließend mit der thrB-Deletionskassette gemischt und mit Quick DNA-Ligase (New England BioLabs, Frankfurt, Deutschland) behandelt.

Der E. coli-Stamm DH5α (Grant et al.; Proceedings of the National Academy of Sciences USA, 87 (1990) 4645 - 4649) wurde dann mit dem Ligationsansatz transformiert (Hanahan, In. DNA Cloning. A Practical Approach. Vol. 1, ILR-Press, Cold Spring Harbor, New York, 1989). Die Selektion von Plasmid tragenden Zellen wurde bei 30°C durchgeführt, indem der Transformationsansatz auf LB-Agar plattiert wurde (Sambrook et al., Molecular Cloning: A Laboratory Manual. 2. Ausgabe, Cold Spring Harbor, New York, 1989), welcher mit 20 mg/l Chloramphenicol ergänzt wurde.

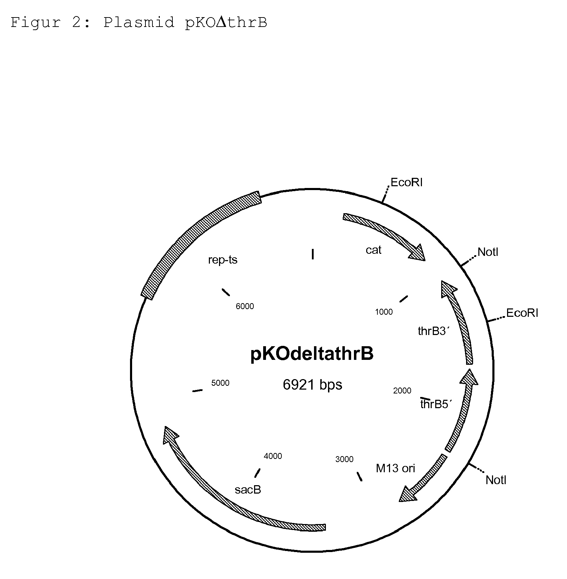

Plasmid-DNA wurde aus einem Transformanten mit Hilfe des QIAprep Spin Miniprep-Kits von Qiagen isoliert und durch eine Restriktionsspaltung mit dem Enzym EcoRI und eine nachfolgende Agarose-Gelelektrophorese überprüft. Das Plasmid wird als pKOΔthrB (Figur 2) bezeichnet.

### 3.2 Sequenzspezifische Mutagenese des thrB-Gens beim E. coli-Stamm MG442

Der L-Threonin produzierende E. coli-Stamm MG442 wird in der Patentschrift US-A- 4 278 765 beschrieben und ist als CMIM B-1628 bei der Russischen Nationalen Sammlung für Industrielle Mikroorganismen (VKPM, Moskau, Russland) eingereicht.

Für den Austausch des chromosomalen thrB-Gens durch das Plasmid-kodierte Deletions-Konstrukt wurde MG442 mit dem Plasmid pKOΔthrB transformiert. Der Genaustausch wurde durch das Selektionsverfahren, das von Link et al. (Journal of Bacteriology 179: 6228-6237 (1997)) beschrieben wurde, durchgeführt und wurde durch Standard-PCR-Verfahren (Innis et al. (1990) PCR Protocols. A guide to methods and applications, Academic Press) mit den folgenden Oligonukleotid-Primern verifiziert:
thrB_No: 5' - CAGTACGCGGCCGCTGCAGGTGATGAATTGATGAAG - 3' (SEQ ID No. 11)
thrB_Co: 5' - TAGTCGCGGCCGCGCCTCGTGGATAGAGGATAACC - 3' (SEQ ID No. 14)

Nach dem Austausch war die in SEQ ID No. 15 gezeigte Form des ΔthrB-Allels in MG442 vorhanden. Der erhaltene Stamm wird als MG422ΔthrB bezeichnet.

### Beispiel 4

### In-vivo-Mutagenese des Stamms MG422ΔthrB

Ausgehend von MG422ΔthrB wurden nach einer Inkubation bei 37°C in L-Homoserin (Sigma, Taufkirchen, Deutschland) enthaltendem Minimal-Agar, zu welchem 2 g/l Glucose und 2 g/l Threonin und Isoleucin hinzugefügt wurden, spontane Mutanten isoliert, die in der Lage waren, bei 2 g/l L-Homoserin zu wachsen. Einem selektierten Mutanten wurde der Name MG422ΔthrBHom2 gegeben.

### Beispiel 5

### Konstruktion des Expressions-Plasmids pTrc99AthrA

Das thrA-Gen aus E. coli K12 wurde unter Verwendung der Polymerasekettenreaktion (PCR) und synthetischer Oligonukleotide amplifiziert. Ausgehend von der Nukleotidsequenz des thrA-Gens in E. coli K12 MG1655 (Zugangsnummer U00096, Blattner et al. (Science 277: 1453-1462 (1997)), wurden PCR-Primer synthetisiert (MWG Biotech, Ebersberg, Deutschland):
thrA1: 5` - AAGGTAACGAGGTAACAACC - 3' (SEQ ID No. 16)
thrA2: 5' - TCAAACCCGACGCTCATATT - 3' (SEQ ID No. 17)

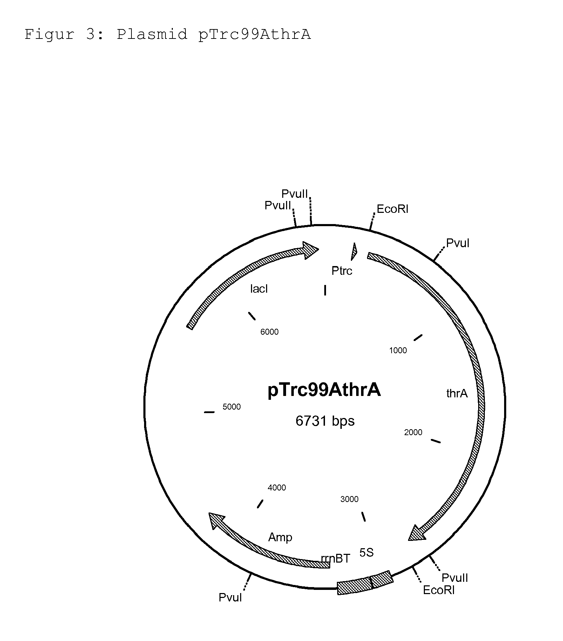

Die für die PCR verwendete chromosomale E. coli K12 MG1655 DNA wurde gemäß den Anweisungen des Herstellers mit "Qiagen Genomic-tips 100/G" (QIAGEN, Hilden, Deutschland) isoliert. Ein DNA-Fragment, ca. 2537 bp groß, konnte mit den spezifischen Primern unter Standard-PCR-Bedingungen (Innis et al. (1990) PCR Protocols. A Guide to Methods and Applications, Academic Press) mit der Pfu-DNA-Polymerase (Promega Corporation, Madison, USA) amplifiziert werden. Das PCR-Produkt wurde mit dem Vektor pCR-Blunt II-TOPO (Zero Blunt TOPO PCR Cloning Kit, Invitrogen, Groningen, Niederlande) gemäß den Anweisungen des Herstellers ligiert und in den E. coli-Stamm TOP10 transformiert. Eine Selektionierung nach Plasmid tragenden Zellen wurde auf LB-Agar durchgeführt, dem 50 µg/ml Kanamycin hinzugesetzt wurden. Nach der Isolierung der Plasmid-DNA wurde der Vektor pCR-Blunt II-TOPO-thrA mit dem Restriktions-Enzym EcoRI gespalten, und nach der Trennung in 0,8%-igem Agarosegel mit Hilfe des QIAquick Gel Extraction Kits (QIAGEN, Hilden, Deutschland) wurde das thrA-Fragment isoliert. Der Vektor pTrc99A (Pharmacia Biotech, Uppsala, Schweden) wurde mit dem Enzym EcoRI gespalten und mit dem isolierten thrA-Fragment ligiert. Der E. coli-Stamm XL1-Blue MRF` (Stratagene, La Jolla, USA) wurde mit dem Ligationsansatz transformiert, und Plasmid tragende Zellen wurden auf LB-Agar selektioniert, dem 50 µg/ml Ampicillin zugesetzt wurden. Eine erfolgreiche Klonierung konnte nach der Isolierung von Plasmid-DNA durch eine Kontrollspaltung mit den Enzymen PvuI und PvuII nachgewiesen werden. Das Plasmid wird als pTrc99AthrA (Figur 3) bezeichnet.

### Beispiel 6

### Herstellung von L-Homoserin mit dem Stamm MG442ΔthrBHom2/pTrc99AthrA/pMW218ytfF

Der Stamm MG442ΔthrBHom2 wurde mit den in Beispiel 5 und 1 beschriebenen Expressions-Plasmiden pTrc99AthrA und pMW218ytfF in zwei aufeinander folgenden Schritten transformiert und Plasmid tragende Zellen wurden auf LB-Agar mit 50 µg/ml Ampicillin oder 50 µg/ml Ampicillin und 50 µg/ml Kanamycin selektioniert. Auf diese Weise bildeten sich die Stämme MG442ΔthrBHom2/pTrc99AthrA und MG442ΔthrBHom2/pTrc99AthrA/pMW218ytfF. Ausgewählte einzelne Kolonien wurden dann auf Minimalmedium mit der folgenden Zusammensetzung weiter vermehrt: 3,5 g/l Na₂HPO₄*2H₂O, 1,5 g/l KH₂PO₄, 1 g/l NH₄Cl, 0,1 g/l MgSO₄*7H₂O, 2 g/l Glucose, 20 g/l Agar, 50 mg/l Ampicillin (oder 50 mg/l Ampicillin und 50 mg/l Kanamycin), 10 mg/l L-Threonin, 10 mg/l L-Isoleucin.

Die Bildung von L-Homoserin durch die Stämme MG442ΔthrBHom2/pTrc99AthrA und MG442ΔthrBHom2/pTrc99AthrA/pMW218ytfF wurde in Batch-Kulturen von 10 ml überprüft, die in 100 ml Erlenmeyerkolben enthalten waren. Dazu wurden 10 ml des Vorkulturmediums der folgenden Zusammensetzung: 2 g/l Hefeextrakt, 10 g/l (NH₄)₂SO₄, 1 g/l KH₂PO₄, 0,5 g/l MgSO₄*7H₂O, 15 g/l CaCO₃, 20 g/l Glucose und 50 mg/l Ampicillin (oder 50 mg/l Ampicillin und 50 mg/l Kanamycin) angeimpft, und der Batch wurde 16 Stunden lang bei 37°C und 180 U/min auf einem ESR-Inkubator von der Kühner AG (Birsfelden, Schweiz) inkubiert. 250 µl dieser Vorkultur wurden in 10 ml Produktionsmedium (25 g/l (NH₄)₂SO₄, 2 g/l KH₂PO₄, 1 g/l MgSO₄*7H₂O, 0,03 g/l FeSO₄*7H₂O, 0,018 g/l MnSO₄*1H₂O, 30 g/l CaCO₃, 20 g/l Glucose, 50 mg/l Ampicillin (oder 50 mg/l Ampicillin und 50 mg/l Kanamycin), 10 mg/l L-Threonin, 10 mg/l L-Isoleucin und 5 mg/l Thiamin) überimpft, und der Batch wurde 72 Stunden lang bei 37°C inkubiert. Nach der Inkubation wurde die optische Dichte (OD) der Kultursuspension mit einem LP2W-Photometer von Dr. Lange (Berlin, Deutschland) bei einer Messwellenlänge von 660 nm bestimmt.

Die Konzentration an gebildetem L-Homoserin wurde dann im steril filtrierten Kulturüberstand mit einem Aminosäureanalysator von Eppendorf-BioTronik (Hamburg, Deutschland) durch Ionenaustauschchromatographie und Nachsäulen-Reaktion mit Ninhydrindetektion bestimmt.

Das Ergebnis des Experiments ist in Tabelle 2 dargestellt.

**Tabelle 2**

| Stamm | OD (660 nm) | L-Homoserin g/l |
|---|---|---|
| MG442ΔthrBHom2/pTrc99AthrA | 4,0 | 1,50 |
| MG442ΔthrBHom2/pTrc99AthrA/pMW218ytfF | 3,8 | 1,85 |

Kurze Beschreibung der Figur:
• Figur 1: pMW218ytfF
• Figur 2: pKOΔthrB (= pKOdeltathrB)
• Figur 3: pTrc99AthrA

Die Längenangaben sollen als ca.-Angaben verstanden werden. Die verwendeten Abkürzungen und Bezeichnungen haben die folgende Bedeutung:
• Amp: Ampicillin-Resistenzgen
• cat: Chloramphenicol-Resistenzgen
• Kan: Kanamycin-Resistenzgen
• lacI: Gen für das Repressor-Protein des trc-Promotors
• Ptrc: trc-Promotorregion, IPTG-induzierbar
• thrA: Kodierende Region des thrA-Gens
• 5S: 5S rRNA-Region
• rrnBT: rRNA-Terminatorregion
• M13 ori: Replikationsstartpunkt
• sacB: sacB-Gen
• thrB5': Teil der 5'-Region des thrB-Gens und der stromaufwärts liegenden Region
• thrB3': Teil der 3'-Region des thrB-Gens und der stromabwärts liegenden Region
• ytfF5': Teil der 5'-Region des ytfF-ORF und der stromaufwärts liegenden Region
• YtfF3': Teil der 3'-Region des ytfF-ORF und der stromabwärts liegenden Region

Die Abkürzungen für die Restriktions-Enzyme haben die folgende Bedeutung
• EcoRI: Restriktions-Endonuclease aus Escherichia coli RY13
• PvuI: Restriktions-Endonuclease aus Proteus vulgaris
• MluI: Restriktions-Endonuclease aus Micrococcus luteus
• NotI: Restriktions-Endonuclease aus Nocardia otitidiscaviarum
• BamHI: Restriktions-Endonuclease aus Bacillus amyloliquefaciens H

S E Q U E N Z A U F L I S T U N G

<110> Degussa GmbH

<120> Verfahren zur Herstellung von L-Aminosäuren unter Verwendung von
      Stämmen der Enterobacteriaceae-Familie

<130> xxx

<160> 17

<170> PatentInhaberin Version 3.3

<210> 1
<211> 975
<212> DNA
<213> Escherichia coli


<220>
<221> CDS
<222> (1)..(975)
<223> ytfF - Codierregion

<400> 1
atg cca gta atg att agc ggc gtg ctg tac gcc ctg tta gca ggg ttg      48
Met Pro Val Met Ile Ser Gly Val Leu Tyr Ala Leu Leu Ala Gly Leu
1               5                   10                  15

atg tgg ggg ctt att ttt gtc ggg ccg ttg atc gtg ccg gaa tac ccg      96
Met Trp Gly Leu Ile Phe Val Gly Pro Leu Ile Val Pro Glu Tyr Pro
            20                  25                  30

gcg atg ttg cag tcg atg ggg cgt tat ctg gcg tta ggg tta att gcg      144
Ala Met Leu Gln Ser Met Gly Arg Tyr Leu Ala Leu Gly Leu Ile Ala
        35                  40                  45

ctg ccc att gcc tgg ctg gga cgc gtg cgt ctg cgt cag ttg gcg cgt      192
Leu Pro Ile Ala Trp Leu Gly Arg Val Arg Leu Arg Gln Leu Ala Arg
    50                  55                  60

cgc gac tgg ctt acc gcc ttg atg ctc act atg atg ggc aac ctc atc      240
Arg Asp Trp Leu Thr Ala Leu Met Leu Thr Met Met Gly Asn Leu Ile
65                  70                  75                  80

tat tac ttc tgc ctt gcc agt gcc att caa cgt act ggc gcg cct gtt      288
Tyr Tyr Phe Cys Leu Ala Ser Ala Ile Gln Arg Thr Gly Ala Pro Val
                85                  90                  95

tcc acg atg att atc ggc acc ctg ccg gtg gtc att cct gtc ttt gcc      336
Ser Thr Met Ile Ile Gly Thr Leu Pro Val Val Ile Pro Val Phe Ala
            100                 105                 110

aat ctg ctt tat agc cag cgc gac ggc aaa ctc gcg tgg gga aaa ctc      384
Asn Leu Leu Tyr Ser Gln Arg Asp Gly Lys Leu Ala Trp Gly Lys Leu
        115                 120                 125

gcc cca gca ctg att tgt att ggc atc ggc ctg gcg tgt gtg aat att      432
Ala Pro Ala Leu Ile Cys Ile Gly Ile Gly Leu Ala Cys Val Asn Ile
    130                 135                 140

gct gag tta aac cac gga ctc ccc gat ttt gac tgg gca cgt tat acc      480

Ala Glu Leu Asn His Gly Leu Pro Asp Phe Asp Trp Ala Arg Tyr Thr
145                 150                 155                 160

tct ggc atc gtg ctg gcg tta gtt tcc gtg gtc tgc tgg gcg tgg tat      528
Ser Gly Ile Val Leu Ala Leu Val Ser Val Val Cys Trp Ala Trp Tyr
                165                 170                 175

gcc ctg cgc aac gcc cgc tgg ctg cgg gaa aat ccc gac aaa cat ccg      576
Ala Leu Arg Asn Ala Arg Trp Leu Arg Glu Asn Pro Asp Lys His Pro
            180                 185                 190

atg atg tgg gca aca gcg cag gcg ctg gtc aca ctg ccg gtt tca ctt      624
Met Met Trp Ala Thr Ala Gln Ala Leu Val Thr Leu Pro Val Ser Leu
        195                 200                 205

atc ggc tat ctc gtc gct tgt tac tgg ctg aat acg caa acg ccg gac      672
Ile Gly Tyr Leu Val Ala Cys Tyr Trp Leu Asn Thr Gln Thr Pro Asp
    210                 215                 220

ttc tcc cta ccc ttt ggc ccc cgt ccg ctg gtg ttt att agt ctg atg      720
Phe Ser Leu Pro Phe Gly Pro Arg Pro Leu Val Phe Ile Ser Leu Met
225                 230                 235                 240

gtt gcg ata gcc gtg ctt tgc tca tgg gtt ggc gca ctc tgc tgg aac      768
Val Ala Ile Ala Val Leu Cys Ser Trp Val Gly Ala Leu Cys Trp Asn
                245                 250                 255

gtc gcc agc cag cta tta ccg aca gtg att ctc ggg ccg ctg att gtt      816
Val Ala Ser Gln Leu Leu Pro Thr Val Ile Leu Gly Pro Leu Ile Val
            260                 265                 270

ttc gaa acg ctg gca ggt ttg ctg tac acc ttt tta ctc cgc cag caa      864
Phe Glu Thr Leu Ala Gly Leu Leu Tyr Thr Phe Leu Leu Arg Gln Gln
        275                 280                 285

atg ccg ccg cta atg acg ctg agc ggt atc gcg ctg tta gtg att ggc      912
Met Pro Pro Leu Met Thr Leu Ser Gly Ile Ala Leu Leu Val Ile Gly
    290                 295                 300

gtg gtc atc gcg gtc aga gca aaa cca gaa aaa cct tta act gaa tct      960
Val Val Ile Ala Val Arg Ala Lys Pro Glu Lys Pro Leu Thr Glu Ser
305                 310                 315                 320

gtc tca gaa agt tga                                                  975
Val Ser Glu Ser

<210>  2
<211>  324
<212>  PRT
<213>  Escherichia coli

<400>  2

Met Pro Val Met Ile Ser Gly Val Leu Tyr Ala Leu Leu Ala Gly Leu
1               5                   10                  15

Met Trp Gly Leu Ile Phe Val Gly Pro Leu Ile Val Pro Glu Tyr Pro
            20                  25                  30

Ala Met Leu Gln Ser Met Gly Arg Tyr Leu Ala Leu Gly Leu Ile Ala
        35                  40                  45

Leu Pro Ile Ala Trp Leu Gly Arg Val Arg Leu Arg Gln Leu Ala Arg
    50                  55                  60

Arg Asp Trp Leu Thr Ala Leu Met Leu Thr Met Met Gly Asn Leu Ile
65                  70                  75                  80

Tyr Tyr Phe Cys Leu Ala Ser Ala Ile Gln Arg Thr Gly Ala Pro Val
                85                  90                  95

Ser Thr Met Ile Ile Gly Thr Leu Pro Val Val Ile Pro Val Phe Ala
            100                 105                 110

Asn Leu Leu Tyr Ser Gln Arg Asp Gly Lys Leu Ala Trp Gly Lys Leu
        115                 120                 125

Ala Pro Ala Leu Ile Cys Ile Gly Ile Gly Leu Ala Cys Val Asn Ile
    130                 135                 140

Ala Glu Leu Asn His Gly Leu Pro Asp Phe Asp Trp Ala Arg Tyr Thr
145                 150                 155                 160

Ser Gly Ile Val Leu Ala Leu Val Ser Val Val Cys Trp Ala Trp Tyr
                165                 170                 175

Ala Leu Arg Asn Ala Arg Trp Leu Arg Glu Asn Pro Asp Lys His Pro
            180                 185                 190

Met Met Trp Ala Thr Ala Gln Ala Leu Val Thr Leu Pro Val Ser Leu
        195                 200                 205

Ile Gly Tyr Leu Val Ala Cys Tyr Trp Leu Asn Thr Gln Thr Pro Asp
    210                 215                 220

Phe Ser Leu Pro Phe Gly Pro Arg Pro Leu Val Phe Ile Ser Leu Met
225                 230                 235                 240

Val Ala Ile Ala Val Leu Cys Ser Trp Val Gly Ala Leu Cys Trp Asn
                245                 250                 255

Val Ala Ser Gln Leu Leu Pro Thr Val Ile Leu Gly Pro Leu Ile Val
            260                 265                 270

Phe Glu Thr Leu Ala Gly Leu Leu Tyr Thr Phe Leu Leu Arg Gln Gln
        275                 280                 285

Met Pro Pro Leu Met Thr Leu Ser Gly Ile Ala Leu Leu Val Ile Gly
    290                 295                 300

Val Val Ile Ala Val Arg Ala Lys Pro Glu Lys Pro Leu Thr Glu Ser
305                 310                 315                 320

Val Ser Glu Ser

<210>  3
<211>  966
<212>  DNA
<213>  Salmonella typhimurium

<220>
<221>  CDS
<222>  (1)..(966)
<223>  ytfF - Codierregion

<400>  3
atg att agc ggc gtg cta tac gcc ctg ctg gca ggg cta atg tgg gga       48
Met Ile Ser Gly Val Leu Tyr Ala Leu Leu Ala Gly Leu Met Trp Gly
1               5                   10                  15

ttg att ttc gtc ggc ccg tta atc gtg ccg gat tac ccg gcc ctc tta       96
Leu Ile Phe Val Gly Pro Leu Ile Val Pro Asp Tyr Pro Ala Leu Leu
            20                  25                  30

cag tca atg gga cgc tat ctg gcg ctg ggg ctg att gcc ctg ccg att      144
Gln Ser Met Gly Arg Tyr Leu Ala Leu Gly Leu Ile Ala Leu Pro Ile
        35                  40                  45

gcc tgg tta ggc cgc acg cgg tta cgc cag tta acg cgc aaa gac tgg      192
Ala Trp Leu Gly Arg Thr Arg Leu Arg Gln Leu Thr Arg Lys Asp Trp
    50                  55                  60

tcc acc gcc ctg tcg ctg acc ctg atg ggc aat gta atc tat tac gcc      240
Ser Thr Ala Leu Ser Leu Thr Leu Met Gly Asn Val Ile Tyr Tyr Ala
65                  70                  75                  80

tgc ctc gcc agc gcc att cag cga acc ggc gcg ccg gtg tcg acg atg      288
Cys Leu Ala Ser Ala Ile Gln Arg Thr Gly Ala Pro Val Ser Thr Met
                85                  90                  95

atc atc ggg aca ttg ccg gtc gtc att ccg ctt ttc gct aac gtt ctc      336
Ile Ile Gly Thr Leu Pro Val Val Ile Pro Leu Phe Ala Asn Val Leu
            100                 105                 110

tat agc caa cga gac ggc aga tta tcc tgg cgg cgt tta gcg ccc gcg      384
Tyr Ser Gln Arg Asp Gly Arg Leu Ser Trp Arg Arg Leu Ala Pro Ala
        115                 120                 125

ctg gcg ctt atc ggt ttt ggc ctg ctg tgc gtc aat att gct gaa ctt      432
Leu Ala Leu Ile Gly Phe Gly Leu Leu Cys Val Asn Ile Ala Glu Leu
    130                 135                 140

agc cag gga ttg tcc gat gtt agc ggc tgg cgc tat ggt tca ggc att      480
Ser Gln Gly Leu Ser Asp Val Ser Gly Trp Arg Tyr Gly Ser Gly Ile
145                 150                 155                 160

gtg ctg gcg ctg att tcc gta gcg tgc tgg gca tgg tat gcg ctg cgc      528
Val Leu Ala Leu Ile Ser Val Ala Cys Trp Ala Trp Tyr Ala Leu Arg
                165                 170                 175

aac gcc cgc tgg ctg cgg gaa aat ccc gac aaa cat ccc atg atg tgg 576
Asn Ala Arg Trp Leu Arg Glu Asn Pro Asp Lys His Pro Met Met Trp
            180                 185                 190

gca acg gcg cag gcg ctg gtt acg ctg ccg gtc tcg tta atc ggc tat 624
Ala Thr Ala Gln Ala Leu Val Thr Leu Pro Val Ser Leu Ile Gly Tyr
        195                 200                 205

gcg gcg gtc tgt ctg tgg ctg agt atc cag aaa ccg ctg ttt acc ctg 672
Ala Ala Val Cys Leu Trp Leu Ser Ile Gln Lys Pro Leu Phe Thr Leu
    210                 215                 220

ccc ttt ggc ccg cat ccc gcg ctg ttt atc ggt ctg atg atc gcc atc 720
Pro Phe Gly Pro His Pro Ala Leu Phe Ile Gly Leu Met Ile Ala Ile
225                 230                 235                 240

gcg gtg ctg tgt tcc tgg gtg ggc gcg ctg tgc tgg aat atc gcc agc 768
Ala Val Leu Cys Ser Trp Val Gly Ala Leu Cys Trp Asn Ile Ala Ser
                245                 250                 255

caa aaa ttg cca acc gtt att tta ggg ccg ctg att gtt ttt gag acg 816
Gln Lys Leu Pro Thr Val Ile Leu Gly Pro Leu Ile Val Phe Glu Thr
            260                 265                 270

ttg gcg gga ctg ctc tat acc ttc att ctg cgc cag gaa atc ccg cca 864
Leu Ala Gly Leu Leu Tyr Thr Phe Ile Leu Arg Gln Glu Ile Pro Pro
        275                 280                 285

ttc ctg acg tta agc ggc att gcg ctc ctg gtg gcg ggc gtg gtc att 912
Phe Leu Thr Leu Ser Gly Ile Ala Leu Leu Val Ala Gly Val Val Ile
    290                 295                 300

gcc gtg aga gcg aaa ccg gaa aaa gcg acc gtc gtt ccg gtc acg gaa 960
Ala Val Arg Ala Lys Pro Glu Lys Ala Thr Val Val Pro Val Thr Glu
305                 310                 315                 320

cgc taa 966
Arg

<210> 4
<211> 321
<212> PRT
<213> Salmonella typhimurium

<400> 4

Met Ile Ser Gly Val Leu Tyr Ala Leu Leu Ala Gly Leu Met Trp Gly
1               5                   10                  15

Leu Ile Phe Val Gly Pro Leu Ile Val Pro Asp Tyr Pro Ala Leu Leu
            20                  25                  30

Gln Ser Met Gly Arg Tyr Leu Ala Leu Gly Leu Ile Ala Leu Pro Ile
        35                  40                  45

Ala Trp Leu Gly Arg Thr Arg Leu Arg Gln Leu Thr Arg Lys Asp Trp
    50                  55                  60

Ser Thr Ala Leu Ser Leu Thr Leu Met Gly Asn Val Ile Tyr Tyr Ala

65                  70                  75                  80

Cys Leu Ala Ser Ala Ile Gln Arg Thr Gly Ala Pro Val Ser Thr Met
                85                  90                  95

Ile Ile Gly Thr Leu Pro Val Val Ile Pro Leu Phe Ala Asn Val Leu
            100                 105                 110

Tyr Ser Gln Arg Asp Gly Arg Leu Ser Trp Arg Arg Leu Ala Pro Ala
        115                 120                 125

Leu Ala Leu Ile Gly Phe Gly Leu Leu Cys Val Asn Ile Ala Glu Leu
    130                 135                 140

Ser Gln Gly Leu Ser Asp Val Ser Gly Trp Arg Tyr Gly Ser Gly Ile
145                 150                 155                 160

Val Leu Ala Leu Ile Ser Val Ala Cys Trp Ala Trp Tyr Ala Leu Arg
                165                 170                 175

Asn Ala Arg Trp Leu Arg Glu Asn Pro Asp Lys His Pro Met Met Trp
            180                 185                 190

Ala Thr Ala Gln Ala Leu Val Thr Leu Pro Val Ser Leu Ile Gly Tyr
        195                 200                 205

Ala Ala Val Cys Leu Trp Leu Ser Ile Gln Lys Pro Leu Phe Thr Leu
    210                 215                 220

Pro Phe Gly Pro His Pro Ala Leu Phe Ile Gly Leu Met Ile Ala Ile
225                 230                 235                 240

Ala Val Leu Cys Ser Trp Val Gly Ala Leu Cys Trp Asn Ile Ala Ser
                245                 250                 255

Gln Lys Leu Pro Thr Val Ile Leu Gly Pro Leu Ile Val Phe Glu Thr
            260                 265                 270

Leu Ala Gly Leu Leu Tyr Thr Phe Ile Leu Arg Gln Glu Ile Pro Pro
        275                 280                 285

Phe Leu Thr Leu Ser Gly Ile Ala Leu Leu Val Ala Gly Val Val Ile
    290                 295                 300

Ala Val Arg Ala Lys Pro Glu Lys Ala Thr Val Val Pro Val Thr Glu
305                 310                 315                 320

Arg

<210>  5
<211>  36
<212>  DNA
<213>  Synthetische Sequenz

<220>
<221>  Primer
<222>  (1)..(36)
<223>  ytfF fw

<400>  5
cagtacggat ccggattggt cgcgataagc catagc                                36

<210>  6
<211>  36
<212>  DNA
<213>  Synthetische Sequenz

<220>
<221>  Primer
<222>  (1)..(36)
<223>  ytfF rev

<400>  6
tagtcaggat ccggattctg acgttggcgc atcgaa                                36

<210>  7
<211>  1352
<212>  DNA
<213>  Escherichia coli

<220>
<221>  CDS
<222>  (238)..(1212)
<223>  PCR-Product mit der ytfF - Codierregion

<400>  7
tagtcaggat ccggattctg acgttggcgc atcgaaaggc ggtctgtttg atgacagcaa      60

aacacttagc aaattgattg gccacccaac gacaacgtta gccgaaagcg taagccatct     120

ttttaatgtt aataactagt taattaaagt ggcatcctcc cgcatcctct ctgataatga     180

cgggatgccg ggagcaatca tgtctgcttc ctgaactttc ttctgacaga tcaatgg        237

atg cca gta atg att agc ggc gtg ctg tac gcc ctg tta gca ggg ttg       285
Met Pro Val Met Ile Ser Gly Val Leu Tyr Ala Leu Leu Ala Gly Leu
1               5                   10                  15

atg tgg ggg ctt att ttt gtc ggg ccg ttg atc gtg ccg gaa tac ccg       333
Met Trp Gly Leu Ile Phe Val Gly Pro Leu Ile Val Pro Glu Tyr Pro
            20                  25                  30

gcg atg ttg cag tcg atg ggg cgt tat ctg gcg tta ggg tta att gcg       381
Ala Met Leu Gln Ser Met Gly Arg Tyr Leu Ala Leu Gly Leu Ile Ala
        35                  40                  45

ctg ccc att gcc tgg ctg gga cgc gtg cgt ctg cgt cag ttg gcg cgt       429

Leu Pro Ile Ala Trp Leu Gly Arg Val Arg Leu Arg Gln Leu Ala Arg
    50                  55                  60

cgc gac tgg ctt acc gcc ttg atg ctc act atg atg ggc aac ctc atc      477
Arg Asp Trp Leu Thr Ala Leu Met Leu Thr Met Met Gly Asn Leu Ile
65                  70                  75                  80

tat tac ttc tgc ctt gcc agt gcc att caa cgt act ggc gcg cct gtt      525
Tyr Tyr Phe Cys Leu Ala Ser Ala Ile Gln Arg Thr Gly Ala Pro Val
                85                  90                  95

tcc acg atg att atc ggc acc ctg ccg gtg gtc att cct gtc ttt gcc      573
Ser Thr Met Ile Ile Gly Thr Leu Pro Val Val Ile Pro Val Phe Ala
            100                 105                 110

aat ctg ctt tat agc cag cgc gac ggc aaa ctc gcg tgg gga aaa ctc      621
Asn Leu Leu Tyr Ser Gln Arg Asp Gly Lys Leu Ala Trp Gly Lys Leu
        115                 120                 125

gcc cca gca ctg att tgt att ggc atc ggc ctg gcg tgt gtg aat att      669
Ala Pro Ala Leu Ile Cys Ile Gly Ile Gly Leu Ala Cys Val Asn Ile
    130                 135                 140

gct gag tta aac cac gga ctc ccc gat ttt gac tgg gca cgt tat acc      717
Ala Glu Leu Asn His Gly Leu Pro Asp Phe Asp Trp Ala Arg Tyr Thr
145                 150                 155                 160

tct ggc atc gtg ctg gcg tta gtt tcc gtg gtc tgc tgg gcg tgg tat      765
Ser Gly Ile Val Leu Ala Leu Val Ser Val Val Cys Trp Ala Trp Tyr
                165                 170                 175

gcc ctg cgc aac gcc cgc tgg ctg cgg gaa aat ccc gac aaa cat ccg      813
Ala Leu Arg Asn Ala Arg Trp Leu Arg Glu Asn Pro Asp Lys His Pro
            180                 185                 190

atg atg tgg gca aca gcg cag gcg ctg gtc aca ctg ccg gtt tca ctt      861
Met Met Trp Ala Thr Ala Gln Ala Leu Val Thr Leu Pro Val Ser Leu
        195                 200                 205

atc ggc tat ctc gtc gct tgt tac tgg ctg aat acg caa acg ccg gac      909
Ile Gly Tyr Leu Val Ala Cys Tyr Trp Leu Asn Thr Gln Thr Pro Asp
    210                 215                 220

ttc tcc cta ccc ttt ggc ccc cgt ccg ctg gtg ttt att agt ctg atg      957
Phe Ser Leu Pro Phe Gly Pro Arg Pro Leu Val Phe Ile Ser Leu Met
225                 230                 235                 240

gtt gcg ata gcc gtg ctt tgc tca tgg gtt ggc gca ctc tgc tgg aac     1005
Val Ala Ile Ala Val Leu Cys Ser Trp Val Gly Ala Leu Cys Trp Asn
                245                 250                 255

gtc gcc agc cag cta tta ccg aca gtg att ctc ggg ccg ctg att gtt     1053
Val Ala Ser Gln Leu Leu Pro Thr Val Ile Leu Gly Pro Leu Ile Val
            260                 265                 270

ttc gaa acg ctg gca ggt ttg ctg tac acc ttt tta ctc cgc cag caa     1101
Phe Glu Thr Leu Ala Gly Leu Leu Tyr Thr Phe Leu Leu Arg Gln Gln
        275                 280                 285

atg ccg ccg cta atg acg ctg agc ggt atc gcg ctg tta gtg att ggc     1149
Met Pro Pro Leu Met Thr Leu Ser Gly Ile Ala Leu Leu Val Ile Gly
    290                 295                 300

gtg gtc atc gcg gtc aga gca aaa cca gaa aaa cct tta act gaa tct     1197
Val Val Ile Ala Val Arg Ala Lys Pro Glu Lys Pro Leu Thr Glu Ser

305                 310                 315                 320

gtc tca gaa agt tga cacgctggca gtgagttaaa taagcctctg ctacgtaagg     1252
Val Ser Glu Ser

gttatagctt ttgccttaaa gatgcattta aaatacatct tatcttatta agaatgaggt   1312

atcagctatg gcttatcgcg accaatccgg atccgtactg                         1352

<210>  8
<211>  324
<212>  PRT
<213>  Escherichia coli

<400>  8

Met Pro Val Met Ile Ser Gly Val Leu Tyr Ala Leu Leu Ala Gly Leu
1               5                   10                  15

Met Trp Gly Leu Ile Phe Val Gly Pro Leu Ile Val Pro Glu Tyr Pro
            20                  25                  30

Ala Met Leu Gln Ser Met Gly Arg Tyr Leu Ala Leu Gly Leu Ile Ala
        35                  40                  45

Leu Pro Ile Ala Trp Leu Gly Arg Val Arg Leu Arg Gln Leu Ala Arg
    50                  55                  60

Arg Asp Trp Leu Thr Ala Leu Met Leu Thr Met Met Gly Asn Leu Ile
65                  70                  75                  80

Tyr Tyr Phe Cys Leu Ala Ser Ala Ile Gln Arg Thr Gly Ala Pro Val
                85                  90                  95

Ser Thr Met Ile Ile Gly Thr Leu Pro Val Val Ile Pro Val Phe Ala
            100                 105                 110

Asn Leu Leu Tyr Ser Gln Arg Asp Gly Lys Leu Ala Trp Gly Lys Leu
        115                 120                 125

Ala Pro Ala Leu Ile Cys Ile Gly Ile Gly Leu Ala Cys Val Asn Ile
    130                 135                 140

Ala Glu Leu Asn His Gly Leu Pro Asp Phe Asp Trp Ala Arg Tyr Thr
145                 150                 155                 160

Ser Gly Ile Val Leu Ala Leu Val Ser Val Val Cys Trp Ala Trp Tyr
                165                 170                 175

Ala Leu Arg Asn Ala Arg Trp Leu Arg Glu Asn Pro Asp Lys His Pro
            180                 185                 190

Met Met Trp Ala Thr Ala Gln Ala Leu Val Thr Leu Pro Val Ser Leu
        195                 200                 205

Ile Gly Tyr Leu Val Ala Cys Tyr Trp Leu Asn Thr Gln Thr Pro Asp
    210                 215                 220

Phe Ser Leu Pro Phe Gly Pro Arg Pro Leu Val Phe Ile Ser Leu Met
225                 230                 235                 240

Val Ala Ile Ala Val Leu Cys Ser Trp Val Gly Ala Leu Cys Trp Asn
                245                 250                 255

Val Ala Ser Gln Leu Leu Pro Thr Val Ile Leu Gly Pro Leu Ile Val
            260                 265                 270

Phe Glu Thr Leu Ala Gly Leu Leu Tyr Thr Phe Leu Leu Arg Gln Gln
        275                 280                 285

Met Pro Pro Leu Met Thr Leu Ser Gly Ile Ala Leu Leu Val Ile Gly
    290                 295                 300

Val Val Ile Ala Val Arg Ala Lys Pro Glu Lys Pro Leu Thr Glu Ser
305                 310                 315                 320

Val Ser Glu Ser

<210>  9
<211>  2006
<212>  DNA
<213>  Escherichia coli

<220>
<221>  CDS
<222>  (594)..(1526)
<223>  thrB - Gen

<400>  9
tgcaggtgat gaattgatga agttctccgg cattctttct ggttcgcttt cttatatctt      60

cggcaagtta gacgaaggca tgagtttctc cgaggcgacc acgctggcgc gggaaatggg     120

ttataccgaa ccggacccgc gagatgatct ttctggtatg gatgtggcgc gtaaactatt     180

gattctcgct cgtgaaacgg gacgtgaact ggagctggcg gatattgaaa ttgaacctgt     240

gctgcccgca gagtttaacg ccgagggtga tgttgccgct tttatggcga atctgtcaca     300

actcgacgat ctctttgccg cgcgcgtggc gaaggcccgt gatgaaggaa aagttttgcg     360

ctatgttggc aatattgatg aagatggcgt ctgccgcgtg aagattgccg aagtggatgg     420

taatgatccg ctgttcaaag tgaaaaatgg cgaaaacgcc ctggccttct atagccacta     480

ttatcagccg ctgccgttgg tactgcgcgg atatggtgcg ggcaatgacg ttacagctgc 540

cggtgtcttt gctgatctgc tacgtaccct ctcatggaag ttaggagtct gac atg 596
Met
1

gtt aaa gtt tat gcc ccg gct tcc agt gcc aat atg agc gtc ggg ttt 644
Val Lys Val Tyr Ala Pro Ala Ser Ser Ala Asn Met Ser Val Gly Phe
            5                   10                  15

gat gtg ctc ggg gcg gcg gtg aca cct gtt gat ggt gca ttg ctc gga 692
Asp Val Leu Gly Ala Ala Val Thr Pro Val Asp Gly Ala Leu Leu Gly
        20                  25                  30

gat gta gtc acg gtt gag gcg gca gag aca ttc agt ctc aac aac ctc 740
Asp Val Val Thr Val Glu Ala Ala Glu Thr Phe Ser Leu Asn Asn Leu
    35                  40                  45

gga cgc ttt gcc gat aag ctg ccg tca gaa cca cgg gaa aat atc gtt 788
Gly Arg Phe Ala Asp Lys Leu Pro Ser Glu Pro Arg Glu Asn Ile Val
50                  55                  60                  65

tat cag tgc tgg gag cgt ttt tgc cag gaa ctg ggt aag caa att cca 836
Tyr Gln Cys Trp Glu Arg Phe Cys Gln Glu Leu Gly Lys Gln Ile Pro
                70                  75                  80

gtg gcg atg acc ctg gaa aag aat atg ccg atc ggt tcg ggc tta ggc 884
Val Ala Met Thr Leu Glu Lys Asn Met Pro Ile Gly Ser Gly Leu Gly
            85                  90                  95

tcc agt gcc tgt tcg gtg gtc gcg gcg ctg atg gcg atg aat gaa cac 932
Ser Ser Ala Cys Ser Val Val Ala Ala Leu Met Ala Met Asn Glu His
        100                 105                 110

tgc ggc aag ccg ctt aat gac act cgt ttg ctg gct ttg atg ggc gag 980
Cys Gly Lys Pro Leu Asn Asp Thr Arg Leu Leu Ala Leu Met Gly Glu
    115                 120                 125

ctg gaa ggc cgt atc tcc ggc agc att cat tac gac aac gtg gca ccg 1028
Leu Glu Gly Arg Ile Ser Gly Ser Ile His Tyr Asp Asn Val Ala Pro
130                 135                 140                 145

tgt ttt ctc ggt ggt atg cag ttg atg atc gaa gaa aac gac atc atc 1076
Cys Phe Leu Gly Gly Met Gln Leu Met Ile Glu Glu Asn Asp Ile Ile
                150                 155                 160

agc cag caa gtg cca ggg ttt gat gag tgg ctg tgg gtg ctg gcg tat 1124
Ser Gln Gln Val Pro Gly Phe Asp Glu Trp Leu Trp Val Leu Ala Tyr
            165                 170                 175

ccg ggg att aaa gtc tcg acg gca gaa gcc agg gct att tta ccg gcg 1172
Pro Gly Ile Lys Val Ser Thr Ala Glu Ala Arg Ala Ile Leu Pro Ala
        180                 185                 190

cag tat cgc cgc cag gat tgc att gcg cac ggg cga cat ctg gca ggc 1220
Gln Tyr Arg Arg Gln Asp Cys Ile Ala His Gly Arg His Leu Ala Gly
    195                 200                 205

ttc att cac gcc tgc tat tcc cgt cag cct gag ctt gcc gcg aag ctg 1268
Phe Ile His Ala Cys Tyr Ser Arg Gln Pro Glu Leu Ala Ala Lys Leu
210                 215                 220                 225

atg aaa gat gtt atc gct gaa ccc tac cgt gaa cgg tta ctg cca ggc 1316
Met Lys Asp Val Ile Ala Glu Pro Tyr Arg Glu Arg Leu Leu Pro Gly
                230                 235                 240

ttc cgg cag gcg cgg cag gcg gtc gcg gaa atc ggc gcg gta gcg agc     1364
Phe Arg Gln Ala Arg Gln Ala Val Ala Glu Ile Gly Ala Val Ala Ser
            245                 250                 255

ggt atc tcc ggc tcc ggc ccg acc ttg ttc gct ctg tgt gac aag ccg     1412
Gly Ile Ser Gly Ser Gly Pro Thr Leu Phe Ala Leu Cys Asp Lys Pro
        260                 265                 270

gaa acc gcc cag cgc gtt gcc gac tgg ttg ggt aag aac tac ctg caa     1460
Glu Thr Ala Gln Arg Val Ala Asp Trp Leu Gly Lys Asn Tyr Leu Gln
    275                 280                 285

aat cag gaa ggt ttt gtt cat att tgc cgg ctg gat acg gcg ggc gca     1508
Asn Gln Glu Gly Phe Val His Ile Cys Arg Leu Asp Thr Ala Gly Ala
290                 295                 300                 305

cga gta ctg gaa aac taa atgaaactct acaatctgaa agatcacaac            1556
Arg Val Leu Glu Asn
                310

gagcaggtca gctttgcgca agccgtaacc caggggttgg gcaaaaatca ggggctgttt   1616

tttccgcacg acctgccgga attcagcctg actgaaattg atgagatgct gaagctggat   1676

tttgtcaccc gcagtgcgaa gatcctctcg gcgtttattg gtgatgaaat cccacaggaa   1736

atcctggaag agcgcgtgcg cgcggcgttt gccttcccgg ctccggtcgc caatgttgaa   1796

agcgatgtcg gttgtctgga attgttccac gggccaacgc tggcatttaa agatttcggc   1856

ggtcgcttta tggcacaaat gctgacccat attgcgggtg ataagccagt gaccattctg   1916

accgcgacct ccggtgatac cggagcggca gtggctcatg ctttctacgg tttaccgaat   1976

gtgaaagtgg ttatcctcta tccacgaggc                                    2006

<210>  10
<211>  310
<212>  PRT
<213>  Escherichia coli

<400>  10

Met Val Lys Val Tyr Ala Pro Ala Ser Ser Ala Asn Met Ser Val Gly
1               5                   10                  15

Phe Asp Val Leu Gly Ala Ala Val Thr Pro Val Asp Gly Ala Leu Leu
            20                  25                  30

Gly Asp Val Val Thr Val Glu Ala Ala Glu Thr Phe Ser Leu Asn Asn
        35                  40                  45

Leu Gly Arg Phe Ala Asp Lys Leu Pro Ser Glu Pro Arg Glu Asn Ile
    50                  55                  60

Val Tyr Gln Cys Trp Glu Arg Phe Cys Gln Glu Leu Gly Lys Gln Ile
65                  70                  75                  80

Pro Val Ala Met Thr Leu Glu Lys Asn Met Pro Ile Gly Ser Gly Leu
                85                  90                  95

Gly Ser Ser Ala Cys Ser Val Val Ala Ala Leu Met Ala Met Asn Glu
            100                 105                 110

His Cys Gly Lys Pro Leu Asn Asp Thr Arg Leu Leu Ala Leu Met Gly
        115                 120                 125

Glu Leu Glu Gly Arg Ile Ser Gly Ser Ile His Tyr Asp Asn Val Ala
    130                 135                 140

Pro Cys Phe Leu Gly Gly Met Gln Leu Met Ile Glu Glu Asn Asp Ile
145                 150                 155                 160

Ile Ser Gln Gln Val Pro Gly Phe Asp Glu Trp Leu Trp Val Leu Ala
                165                 170                 175

Tyr Pro Gly Ile Lys Val Ser Thr Ala Glu Ala Arg Ala Ile Leu Pro
            180                 185                 190

Ala Gln Tyr Arg Arg Gln Asp Cys Ile Ala His Gly Arg His Leu Ala
        195                 200                 205

Gly Phe Ile His Ala Cys Tyr Ser Arg Gln Pro Glu Leu Ala Ala Lys
    210                 215                 220

Leu Met Lys Asp Val Ile Ala Glu Pro Tyr Arg Glu Arg Leu Leu Pro
225                 230                 235                 240

Gly Phe Arg Gln Ala Arg Gln Ala Val Ala Glu Ile Gly Ala Val Ala
                245                 250                 255

Ser Gly Ile Ser Gly Ser Gly Pro Thr Leu Phe Ala Leu Cys Asp Lys
            260                 265                 270

Pro Glu Thr Ala Gln Arg Val Ala Asp Trp Leu Gly Lys Asn Tyr Leu
        275                 280                 285

Gln Asn Gln Glu Gly Phe Val His Ile Cys Arg Leu Asp Thr Ala Gly
    290                 295                 300

Ala Arg Val Leu Glu Asn
305                 310

<210>  11
<211>  36
<212>  DNA
<213>  Escherichia coli

<220>
<221>  Primer
<222>  (1)..(36)
<223>  thrB_No

<400>  11
cagtacgcgg ccgctgcagg tgatgaattg atgaag                                36


<210>  12
<211>  55
<212>  DNA
<213>  Escherichia coli


<220>
<221>  Primer
<222>  (1)..(55)
<223>  thrB_Ni

<400>  12
cacgcaataa ccttcacact ccaaatttat aactaaccat gtcagactcc taact           55


<210>  13
<211>  53
<212>  DNA
<213>  Escherichia coli


<220>
<221>  Primer
<222>  (1)..(53)
<223>  thrB_Ci

<400>  13
gttataaatt tggagtgtga aggttattgc gtgcgacctt gttcgctctg tgt             53


<210>  14
<211>  35
<212>  DNA
<213>  Escherichia coli


<220>
<221>  Primer
<222>  (1)..(35)
<223>  thrB_Co

<400>  14
tagtcgcggc cgcgcctcgt ggatagagga taacc                                 35


<210>  15
<211>  183
<212>  DNA
<213>  Escherichia coli


<220>
<221>  gemischtes Merkmal
<222>  (1)..(183)
<223>  mutagene DNA  delta thrB

<220>
<221>  gemischtes Merkmal
<222>  (1)..(3)
<223>  Start-Codon des delta thrB-Allels

<220>
<221>  gemischtes Merkmal
<222>  (1)..(11)
<223>  5'-Region des delta thrB-Allels

<220>
<221>  gemischtes Merkmal
<222>  (12)..(29)
<223>  technische DNA

<220>
<221>  gemischtes Merkmal
<222>  (30)..(180)
<223>  3'-Region des delta thrB-Allels

<220>
<221>  gemischtes Merkmal
<222>  (181)..(183)
<223>  Stopp-Codon des delta thrB-Allels

<400>  15
atggttagtt ataaatttgg agtgtgaagg ttattgcgtg cgaccttgtt cgctctgtgt     60

gacaagccgg aaaccgccca gcgcgttgcc gactggttgg gtaagaacta cctgcaaaat    120

caggaaggtt ttgttcatat ttgccggctg gatacggcgg gcgcacgagt actggaaaac    180

taa                                                                  183


<210>  16
<211>  20
<212>  DNA
<213>  Escherichia coli


<220>
<221>  Primer
<222>  (1)..(20)
<223>  thrA1

<400>  16
aaggtaacga ggtaacaacc                                                 20


<210>  17
<211>  20
<212>  DNA
<213>  Escherichia coli


<220>
<221>  Primer
<222>  (1)..(20)
<223>  thrA2

<400>  17
tcaaacccga cgctcatatt                                                 20

## Patentansprüche

1. Rekombinante L-Aminosäure produzierende Mikroorganismen, die einen verstärkten oder überexprimierten ytfF-ORF enthalten, der für ein putatives Membranprotein eines Aminosäuretransporters kodiert.

2. Mikroorganismen gemäss Anspruch 1, in denen ein dem ytfF-ORF entsprechendes Polynukleotid verstärkt wird, das für ein Polypeptid kodiert, dessen Aminosäuresequenz zu mindestens 90% identisch ist mit einer Aminosäuresequenz, ausgewählt aus der Gruppe SEQ ID No. 2 und SEQ ID No. 4 .

3. Mikroorganismen gemäss Anspruch 2, **dadurch gekennzeichnet, dass** sie ein dem ytfF-ORF entsprechendes isoliertes Polynukleotid enthalten ausgewählt aus der Gruppe:
a) Polynukleotid mit einer Nukleotidsequenz, ausgewählt aus SEQ ID No. 1 oder SEQ ID No. 3 und den dazu komplementären Nukleotidsequenzen;
b) Polynukleotid mit einer Nukleotidsequenz, die der SEQ ID No. 1 oder SEQ ID No. 3 im Rahmen der Degeneration des genetischen Codes entspricht;
c) Polynukleotidsequenz mit einer Sequenz, die mit der zur Sequenz SEQ ID No. 1 oder SEQ ID No. 3 komplementären Sequenz unter stringenten Bedingungen hybridisiert, wobei die stringenten Bedingungen durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1 oder SEQ ID No. 3, die funktionsneutrale Sinnmutanten enthält,
wobei die Polynukleotide für ein putatives Membranprotein eines Aminosäuretransporters kodieren.

4. Mikroorganismen gemäss Anspruch 2, **dadurch gekennzeichnet, dass** das Polypeptid eine Aminosäuresequenz besitzt, die zu wenigstens 95% identisch ist mit einer der Sequenzen ausgewählt aus der Gruppe SEQ ID No. 2 und SEQ ID No. 4.

5. Mikroorganismen gemäss Anspruch 2, **dadurch gekennzeichnet, dass** das Polypeptid die Aminosäuresequenz aufweist, die 100% identisch ist mit einer der Sequenzen, ausgewählt aus der Gruppe SEQ ID No. 2 oder SEQ ID No. 4.

6. Mikroorganismus gemäss den Ansprüchen 1 bis 5, **dadurch gekennzeichnet, dass** sie durch Transformation, Transduktion, Konjugation oder eine Kombination dieser Methoden hergestellt werden, mit einem Vektor, der den ytfF-ORF, ein Allel dieses ORFs oder Teile davon und/oder einen Promotor enthält.

7. Mikroorganismen gemäss den Ansprüchen 1 bis 6, in denen die Kopienzahl des ytfF-ORFs oder der Allele um mindestens 1 erhöht vorliegt.

8. Mikroorganismen gemäss Anspruch 7, **dadurch gekennzeichnet, dass** die Erhöhung der Kopienzahl des ytfF-ORFs um mindestens 1 durch Integration des ORFs oder der Allele in das Chromosom der Mikroorganismen bewirkt wird.

9. Mikroorganismen gemäss Anspruch 7, **dadurch gekennzeichnet, dass** die Erhöhung der Kopienzahl des ytfF-ORFs um mindestens 1 durch einen extrachromosomal replizierenden Vektor erzielt wird.

10. Mikroorganismen gemäss den Ansprüchen 1 bis 9, **dadurch gekennzeichnet, dass** man zur Erzielung der Verstärkung
a) die Promotor- und Regulationsregion oder die Ribosomenbindungsstelle stromaufwärts des ytfF-ORFs mutiert, oder
b) Expresssionskassetten oder Promotoren stromaufwärts des ytfF-ORFs einbaut.

11. Mikroorganismen gemäss den Ansprüchen 1 bis 9, **dadurch gekennzeichnet, dass** die Expression des ytfF-ORFs unter der Kontrolle eines die Expression des ORFs verstärkenden Promotors steht.

12. Mikroorganismen gemäss den Ansprüchen 1 bis 11, **dadurch gekennzeichnet, dass** durch die Verstärkung des ytfF-ORFs die Konzentration oder Aktivität des ytfF-Genproduktes (Proteins) um mindestens 10% erhöht sind, bezogen auf die Aktivität oder Konzentration des Genproduktes im für den ytfF-ORF nicht rekombinanten Mikroorganismus oder Elternstamm.

13. Mikroorganismen gemäss den Ansprüchen 1 bis 12, **dadurch gekennzeichnet, dass** die Mikroorganismen aus den Gattungen Escherichia, Erwinia, Providencia und Serratia ausgewählt sind.

14. Mikroorganismen gemäss Anspruch 13, **dadurch gekennzeichnet, dass** zusätzlich weitere Gene des Biosyntheseweges der gewünschten L-Aminosäure verstärkt, insbesondere überexprimiert vorliegen.

15. Mikroorganismen gemäss den Ansprüchen 1 bis 14, **dadurch gekennzeichnet, dass** sie L-Threonin oder L-Homoserin produzieren.

16. Verfahren zur Herstellung von L-Aminosäuren durch Fermentation von rekombinanten Mikroorganismen der Familie Enterobacteriaceae, **dadurch gekennzeichnet, dass** man
a) die die gewünschte L-Aminosäure produzierenden Mikroorganismen gemäß den Ansprüchen 1 bis 15 in einem Medium kultiviert unter Bedingungen, bei denen die gewünschte L-Aminosäure im Medium oder in den Zellen angereichert wird, und
b) die gewünschte L-Aminosäure isoliert, wobei Bestandteile der Fermentationsbrühe und/oder die Biomasse in ihrer Gesamtheit oder Anteilen (≥ 0 bis 100%) im isolierten Produkt verbleiben oder vollständig entfernt werden.

17. Verfahren gemäss Anspruch 16 , **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) mindestens ein Gen des für die Aspartatkinase, die Homoserin-Dehydrogenase, die Homoserinkinase und die Threoninsynthase kodierenden thrABC-Operons,
b) das für die Pyruvat-Carboxylase kodierende pyc-Gen von Corynebacterium glutamicum,
c) das für die Phosphoenolpyruvat-Synthase kodierende pps-Gen,
d) das für die Phosphoenolpyruvat-Carboxylase kodierende ppc-Gen,
e) die für die Untereinheiten der Pryridin-Transhydrogenase kodierenden Gene pntA und pntB,
f) das für das Threoninresistenz vermittelnde Protein kodierende Gen rhtC,
g) das für das Threonin-Export-Carrier-Protein kodierende thrE-Gen aus Corynebacterium glutamicum,
h) das für die Glutamat-Dehydrogenase kodierende gdhA-Gen,
i) das für die Phosphohistidin-Protein-Hexose-Phosphotransferase kodierende ptsH-Gen,
j) das für das Enzym I des Phosphotransferase-Systems kodierende ptsI-Gen,
k) das für die Glucose-spezifische IIA Komponente kodierende crr-Gen,
l) das für die Glucose-spezifische IIBC Komponente kodierende ptsG-Gen,
m) das für die Cystein-Synthase A kodierende cysK-Gen,
n) das für den Regulator des cys-Regulons kodierende cysB-Gen,
o) das für das Flavoprotein der NADPH-Sulfit-Reduktase kodierende cysJ-Gen,
p) das für das Hämoprotein der NADPH-Sulfit-Reduktase kodierende cysI-Gen,
q) das für die Adenylylsulfat-Reduktase kodierende cysH-Gen,
r) das für die Decarboxylase Untereinheit der 2-Ketoglutarat Dehydrogenase kodierende sucA-Gen,
s) das für die Dihydrolipoyltranssuccinase E2 Untereinheit der 2-Ketoglutarat Dehydrogenase kodierende sucB-Gen,
t) das für die β-Untereinheit der Succinyl-CoA Synthetase kodierende sucC-Gen,
u) das für die α-Untereinheit der Succinyl-CoA Synthetase kodierende sucD-Gen,
v) das Genprodukt des offenen Leserahmens (ORF) yibD von Escherichia coli,
w) das Genprodukt des offenen Leserahmens (ORF) yjcG von Escherichia coli,
x) das Genprodukt des offenen Leserahmens (ORF) ytfR von Escherichia coli,
y) das Genprodukt des offenen Leserahmens (ORF) ytfT von Escherichia coli,
z) das Genprodukt des offenen Leserahmens (ORF) yjff von Escherichia coli,
aa) das Genprodukt des offenen Leserahmens (ORF) ytfQ von Escherichia coli,
verstärkt, insbesondere überexprimiert. (u.U. weglassen)

18. Verfahren gemäss den Ansprüchen 16 bis 17, **dadurch gekennzeichnet, dass** man Mikroorganismen einsetzt, in denen die Stoffwechselwege zumindest teilweise abgeschwächt sind, die die Bildung der gewünschten L-Aminosäure verringern.

19. Verfahren gemäss Anspruch 18, **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) das für die Threonin-Dehydrogenase kodierende tdh-Gen,
b) das für die Malat-Dehydrogenase kodierende mdh-Gen,
c) das Genprodukt des offenen Leserahmens (ORF) yjfA von Escherichia coli,
d) das Genprodukt des offenen Leserahmens (ORF) ytfP von Escherichia coli,
e) das für die Phosphoenolpyruvat-Carboxykinase kodierende pckA-Gen,
f) das für die Pyruvat-Oxidase kodierende poxB-Gen,
g) das für den DgsA-Regulator des Phosphotransferase-Systems kodierende dgsA-Gen,
h) das für den Fructose-Repressor kodierende fruR-Gen,
i) das für den Sigma³⁸-Faktor kodierende rpoS-Gen, und
j) das für die Aspartat Ammonium-Lyase kodierende aspA-Gen
abschwächt, insbesondere ausschaltet oder die Expression verringert. (u.U. weglassen)

20. Verfahren gemäss den Ansprüchen 16 und 18, **dadurch gekennzeichnet, dass** man L-Aminosäuren, ausgewählt aus der Gruppe L-Asparagin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin herstellt.

21. Verfahren gemäss den Ansprüchen 16 und 18, **dadurch gekennzeichnet, dass** man L-Aminosäuren, ausgewählt aus der Gruppe L-Isoleucin, L-Valin, L-Methionin, L-Homoserin, L-Tryptophan und L-Lysin herstellt.
